# EUROPEAN PATENT APPLICATION

(11) **EP 4 691 440 A1**
(43) Date of publication of application: **11.02.2026**
(21) Application number: 24780940.3
(22) Date of filing: 01.04.2024
(51) Int. Cl.: A61F 13/15

(54) **ABSORBENT BODY MANUFACTURING DEVICE AND MANUFACTURING METHOD**

(30) Priority: 30.03.2023 JP 2023056523
(71) Applicant: Zuiko Corporation, Ibaraki-shi Osaka 5670082 (JP)
(72) Inventor: SHIMADA, Takahiro, Ibaraki-shi, Osaka 567-0082 (JP)
(74) Representative: SSM Sandmair
(86) International application number: PCT/JP2024/013488
(87) International publication number: WO 2024/204848

(57) **Abstract**

Provided are a device and method for manufacturing an absorbent body, with which a hybrid structure absorbent body having a desired shape can be manufactured with high precision. A manufacturing device (1000A) includes an absorbent sheet formation unit (100) that forms an absorbent sheet (Sa) in which a SAP is sprayed between a first sheet (S1) and a second sheet (S2), and presses the absorbent sheet using a press; and a fiber-stacking unit (300) that stacks fibers to form an absorbent fluff (Fa) and stacks the absorbent fluff on the absorbent sheet. When stacking the absorbent fluff on the absorbent sheet, the fiber-stacking unit lays a channel part of the absorbent fluff, where the fibers are not disposed, over a channel area of the absorbent sheet, where the SAP is not sprayed.

## Description

### TECHNICAL FIELD

The present invention relates to an apparatus and method for manufacturing an absorbent body.

### BACKGROUND ART

As shown in Patent Literature 1 (WO 2021/131721 A1), an apparatus and method for manufacturing a hybrid structure absorbent body having a water-absorbing material absorbent layer and a stacked fiber absorbent layer including at least fluff have been known.

Patent Literature 1 (WO 2021/131721 A1) discloses a device and method for manufacturing an absorbent body including a first core made of water-absorbing fibers and a second core in which a SAP serving as a water-absorbing material is disposed between base material sheets.

### PRIOR ART LITERATURE

### PATENT LITERATURE

Patent Literature 1: WO 2021/131721 A1

### SUMMARY OF THE INVENTION

### <Problem to be solved by the invention >

In the device and method for manufacturing the absorbent body disclosed in Patent Literature 1 (WO 2021/131721 A1), the absorbent body is manufactured by disposing a second core made of a SAP on top of a suction-conveyed first core made of water-absorbing fibers. Therefore, the SAP cannot be directly suctioned by a conveyer, and the SAP is likely to move (the SAP is likely to shift positions). Thus, the device and method for manufacturing the absorbent body disclosed in Patent Literature 1 (WO 2021/131721 A1) have room for improvement in terms of manufacturing a hybrid structure absorbent body in a desired shape with high precision.

### <Means for Solving Problem>

An apparatus for manufacturing an absorbent body of one embodiment of the present invention is an apparatus for manufacturing a hybrid structure absorbent body having a water-absorbing material absorbent layer and a stacked fiber absorbent layer including at least fluff. The apparatus for manufacturing the absorbent body comprises an absorbent sheet formation unit and a fiber-stacking unit. The absorbent sheet formation unit is configured to form an absorbent sheet in which an absorbent material is sprayed between a first sheet and a second sheet. The fiber-stacking unit is configured to stack fibers to form an absorbent fluff containing at least fluff, and stack the absorbent fluff on the absorbent sheet, which is conveyed while being sucked. A spraying area and a channel area are formed between the first sheet and the second sheet of the absorbent sheet. The water-absorbing material is sprayed in the spraying area. The water-absorbing material is not sprayed in the channel area. The absorbent fluff has a stacked fiber part and a channel part. Fibers are stacked in the stacked fiber part. Fibers are not disposed in the channel part. The absorbent sheet formation unit has a first sheet supplier, a first suction conveyer, a water-absorbing material supplier, a second sheet supplier, and a press. The first sheet supplier is configured to supply the first sheet. The first suction conveyer includes a first conveying surface having a suction function, and is configured to convey the first sheet while suctioning the first sheet on the first conveying surface. The water-absorbing material supplier is configured to supply the water-absorbing material onto the first sheet conveyed by the first suction conveyer. The second sheet supplier is configured to supply the second sheet and cover the water-absorbing material on the first sheet with the second sheet to form the absorbent sheet. The press is configured to press the conveyed absorbent sheet. The fiber-stacking unit has a fiber supplier, a fiber-stacking drum, and a third sheet supplier. The fiber supplier is configured to supply the fibers. The fiber-stacking drum has a suction function and is configured to cause the fibers supplied by the fiber supplier to be stacked on a peripheral surface of the fiber-stacking drum to form the absorbent fluff. The third sheet supplier is configured to supply a third sheet to be disposed on a side of the absorbent fluff opposite from the absorbent sheet such that the absorbent fluff is sandwiched between the third sheet and the absorbent sheet. The fiber-stacking unit is configured to lay the channel part of the absorbent fluff over the channel area of the absorbent sheet when stacking the absorbent fluff on the absorbent sheet.

A method for manufacturing an absorbent body of one embodiment of the present invention is a method for manufacturing a hybrid structure absorbent body having a water-absorbing material layer and a stacked fiber layer. The method for manufacturing the absorbent body comprises an absorbent sheet formation process, a pressing process, an absorbent fluff formation process, and a stacking process. In the absorbent sheet formation process, a water-absorbing material is sprayed between a first sheet and a second sheet, and an absorbent sheet is formed. A spraying area in which the water-absorbing material is sprayed and a channel area in which the water-absorbing material is not sprayed are formed between the first sheet and the second sheet of the absorbent sheet. In the pressing process, the conveyed absorbent sheet is pressed. In the absorbent fluff formation process, an absorbent fluff, which has a stacked fiber part in which fibers are stacked and a channel part in which the fibers are not stacked, is formed. In the stacking process, the absorbent fluff is stacked on top of the absorbent sheet, which is conveyed while being sucked. In the stacking process, the channel part of the absorbent fluff is laid over the channel area of the absorbent sheet.

### <Effects of Invention>

In the apparatus and method for manufacturing a wearable article of the present invention, the absorbent sheet, in which the water-absorbing material is likely to shift position, is directly conveyed by suction by a suction conveyer, and the absorbent fluff is laid over the absorbent sheet. Therefore, in the device and method for manufacturing a wearable article of the present invention, the fluidity of the water-absorbing material can be suppressed, differently from what may happen when the absorbent body is manufactured by disposing the absorbent sheet on the suction-conveyed water-absorbing fibers, and a hybrid structure absorbent body in a desired shape can be manufactured with high precision.

### BRIEF DESCRIPTION OF THE DRAWINGS

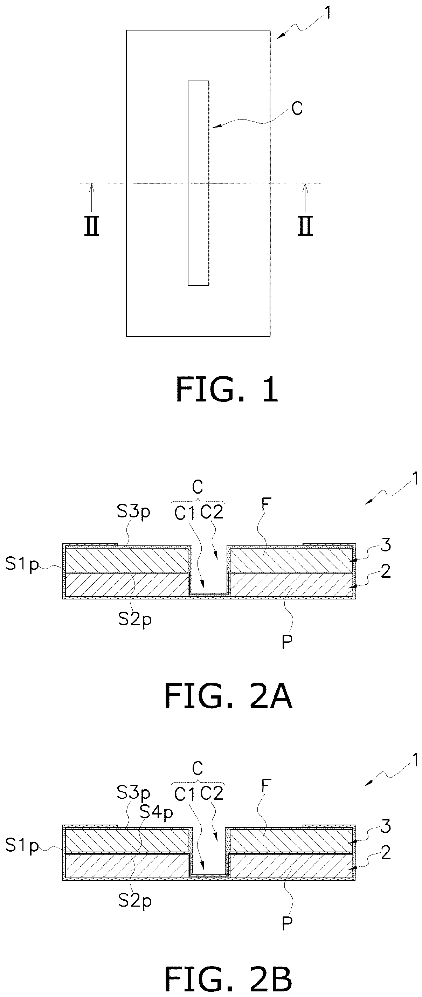

FIG. 1 is a schematic plan view of an absorbent body manufactured using the manufacturing device and manufacturing method of the present invention.
FIG. 2A is a cross-sectional view, taken along line II-II in FIG. 1, of a case in which the absorbent body of FIG. 1 includes three sheets.
FIG. 2B
   is a cross-sectional view, taken along line II-II in FIG. 1, of a case in which the absorbent body of FIG. 1 includes four sheets.

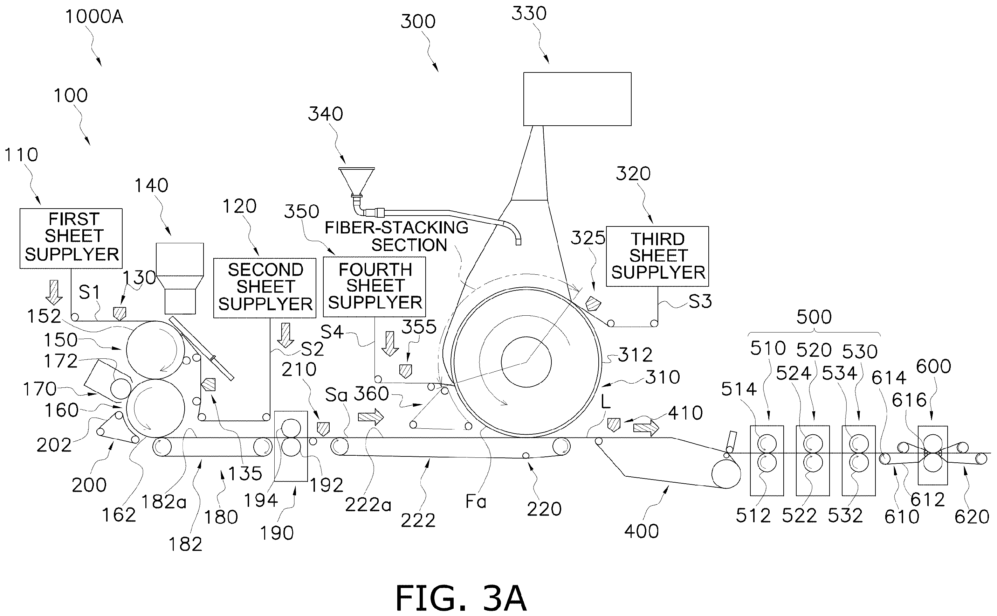

FIG. 3A is a schematic process diagram of an apparatus for manufacturing an absorbent body according to a first embodiment of the present invention.

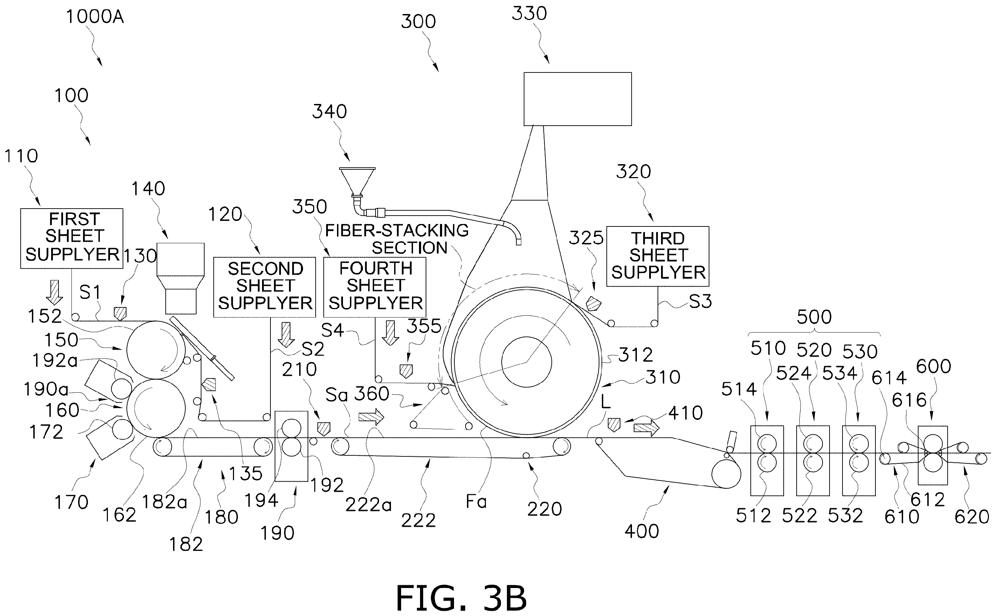

FIG. 3B is a schematic process diagram of an apparatus for manufacturing an absorbent body according to another example of the first embodiment of the present invention.

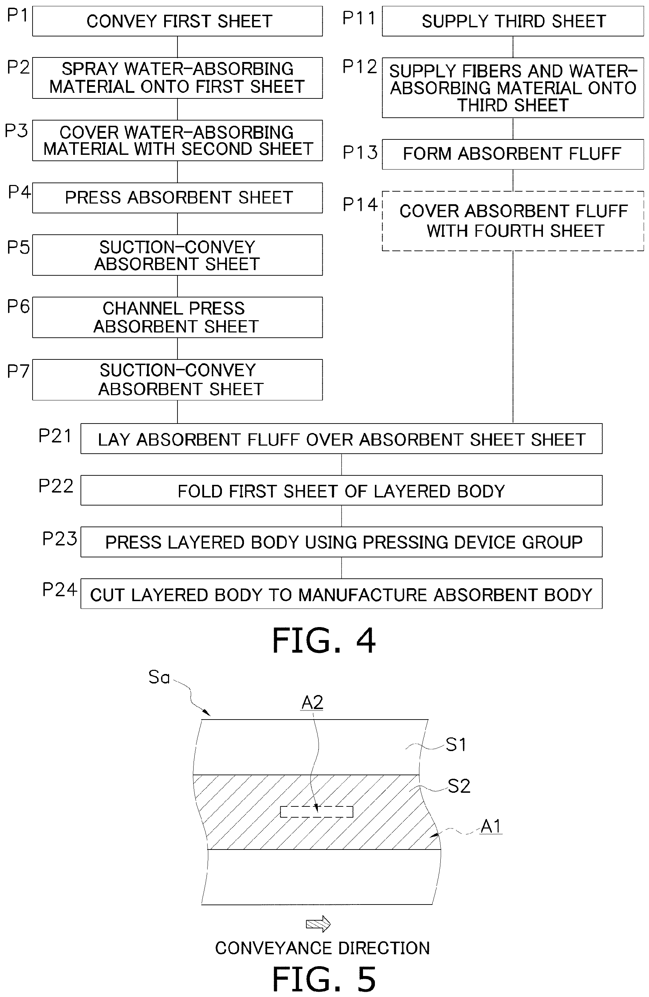

FIG. 4 is a schematic flowchart of a method for manufacturing an absorbent body according to the first embodiment of the present invention.
FIG. 5 is a view of an absorbent sheet conveyed by a third suction conveyer of an absorbent sheet formation unit of the manufacturing device of FIG. 3, viewed along a thickness direction of the absorbent sheet.

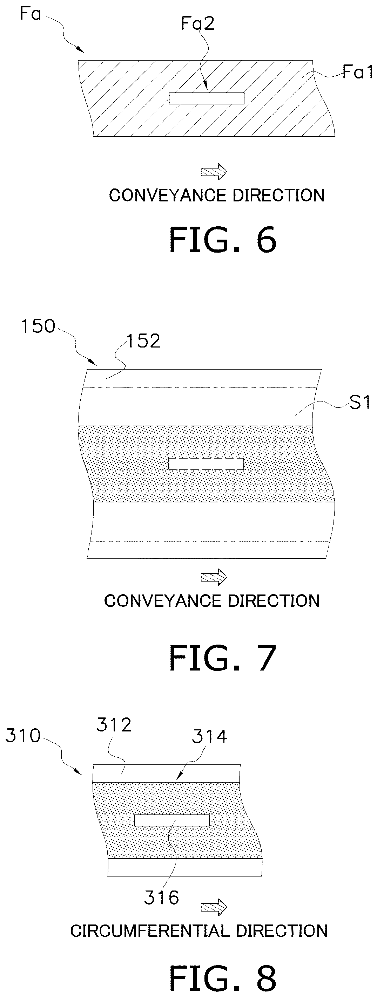

FIG. 6 is a view of an absorbent fluff formed by a fiber-stacking unit of the manufacturing device of FIG. 3, viewed along a thickness direction of the absorbent fluff.
FIG. 7 is a diagram schematically illustrating a conveying surface of a first drum of the absorbent sheet formation unit of the manufacturing device of FIG. 3.
FIG. 8 is a diagram schematically illustrating an outer peripheral surface of a fiber-stacking drum of the fiber-stacking unit of the manufacturing device of FIG. 3.

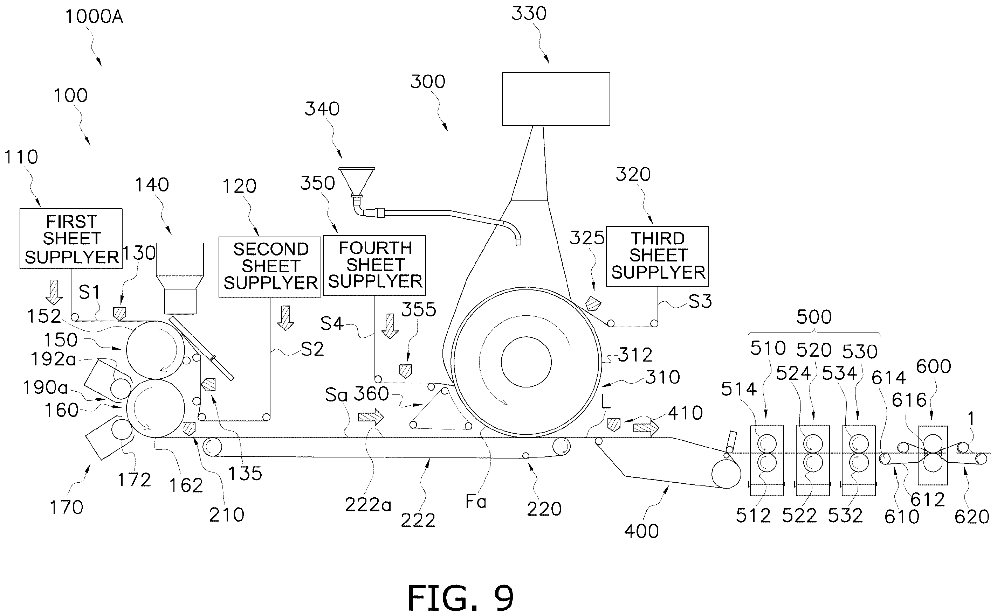

FIG. 9 is a schematic process diagram of an apparatus for manufacturing an absorbent body according to a modification B of the first embodiment of the present invention.

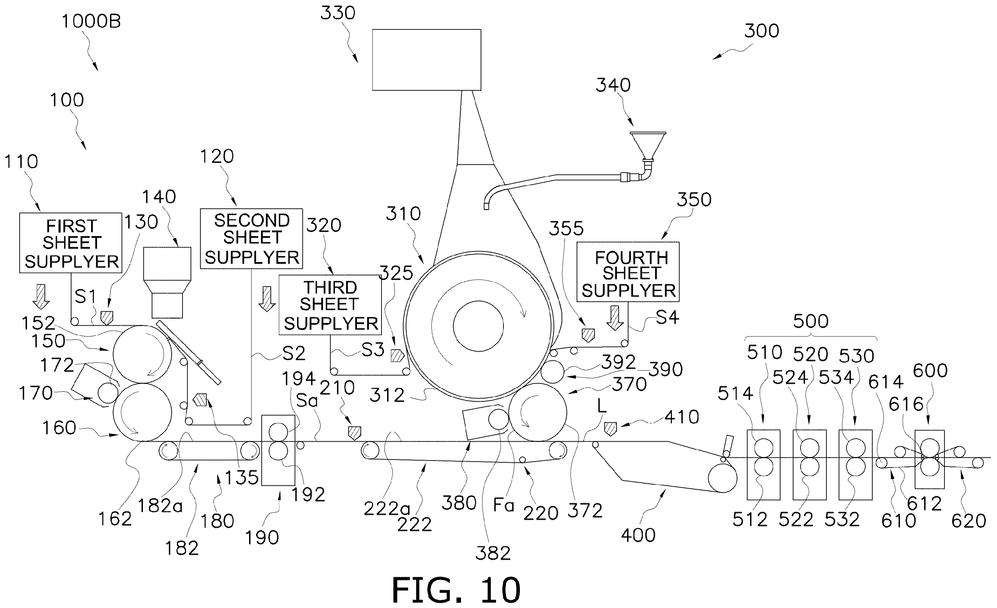

FIG. 10 is a schematic process diagram of an apparatus for manufacturing an absorbent body according to a second embodiment of the present invention.

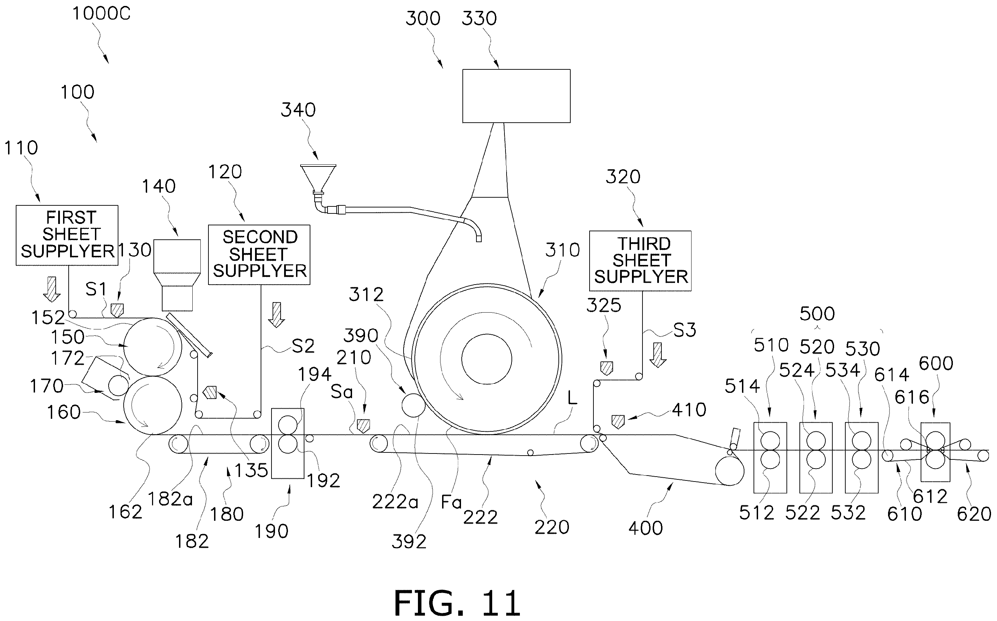

FIG. 11 is a schematic process diagram of an apparatus for manufacturing an absorbent body according to a third embodiment of the present invention.

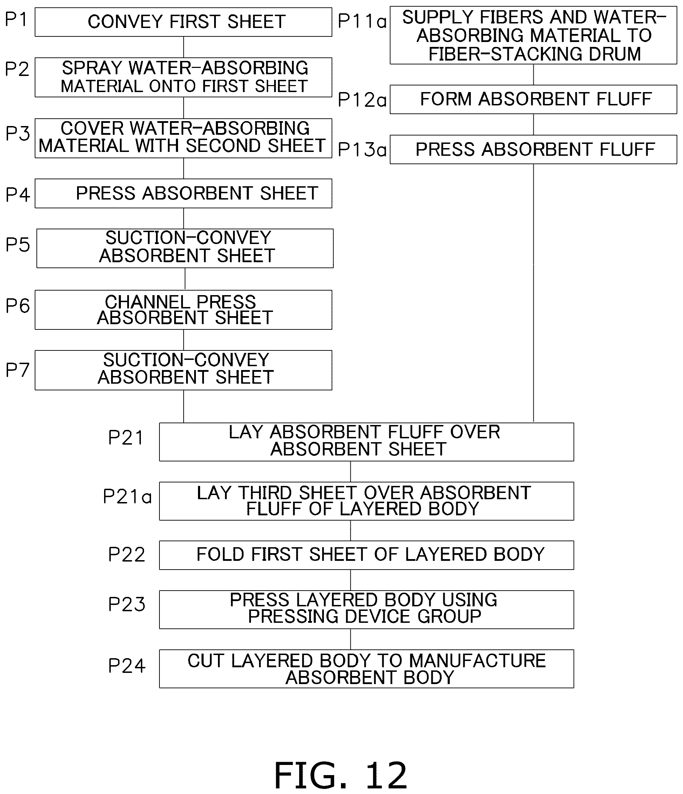

FIG. 12 is a schematic flowchart of a method for manufacturing an absorbent body according to the third embodiment of the present invention.

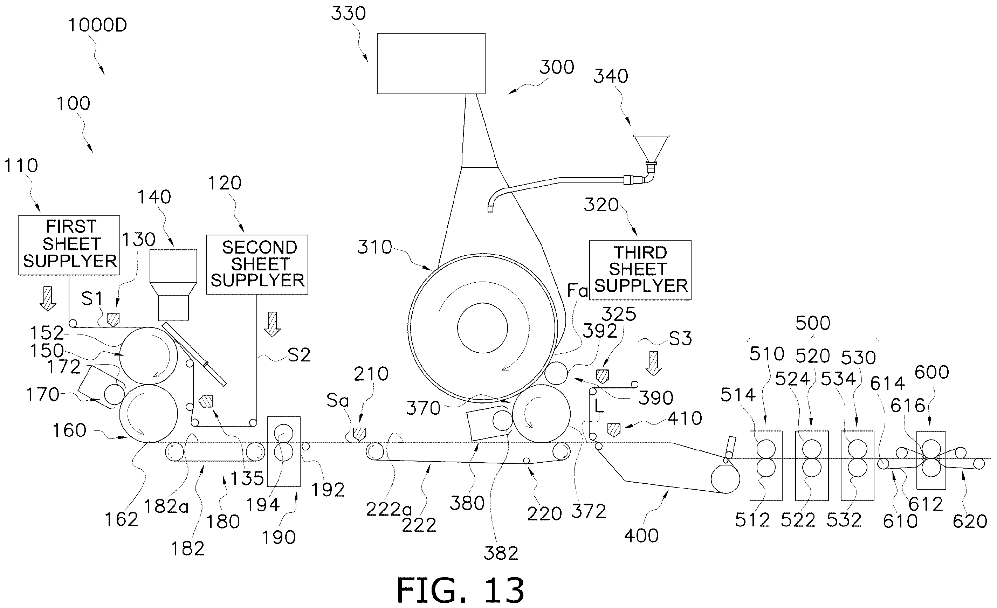

FIG. 13 is a schematic process diagram of an apparatus for manufacturing an absorbent body according to a fourth embodiment of the present invention.

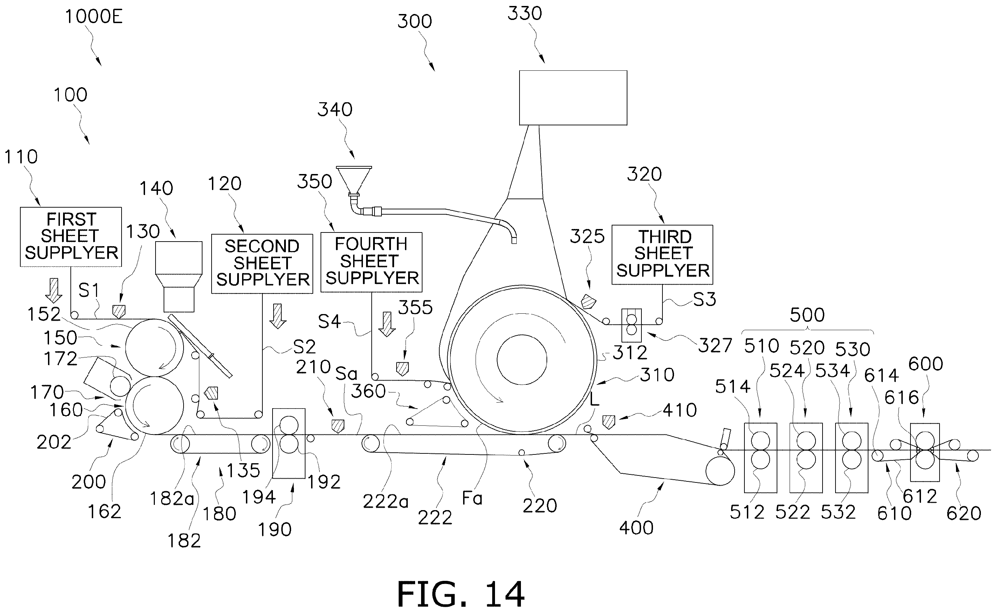

FIG. 14 is a schematic process diagram of an apparatus for manufacturing an absorbent body according to a fifth embodiment of the present invention.

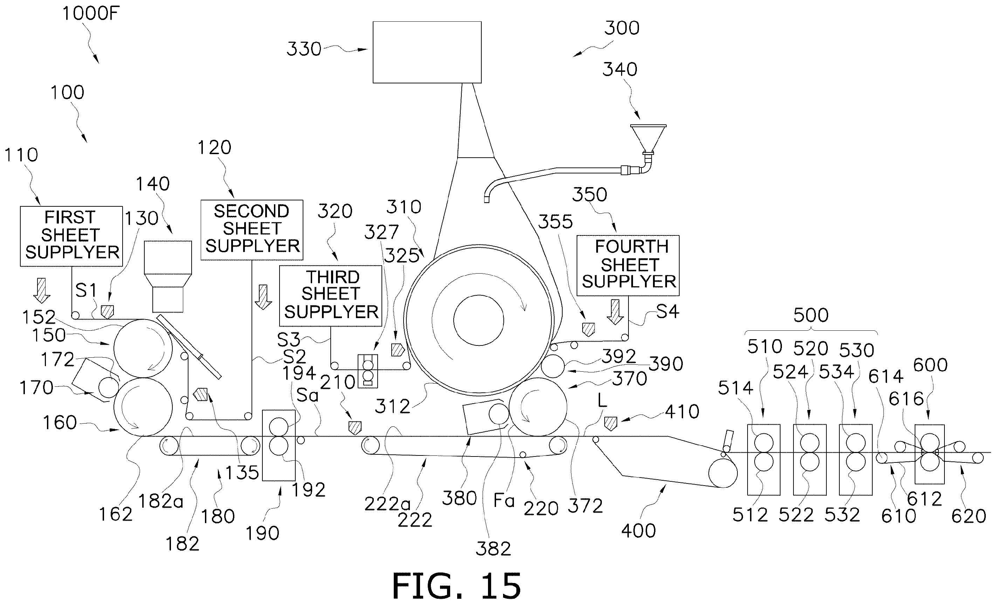

FIG. 15 is a schematic process diagram of an apparatus for manufacturing an absorbent body according to a sixth embodiment of the present invention.

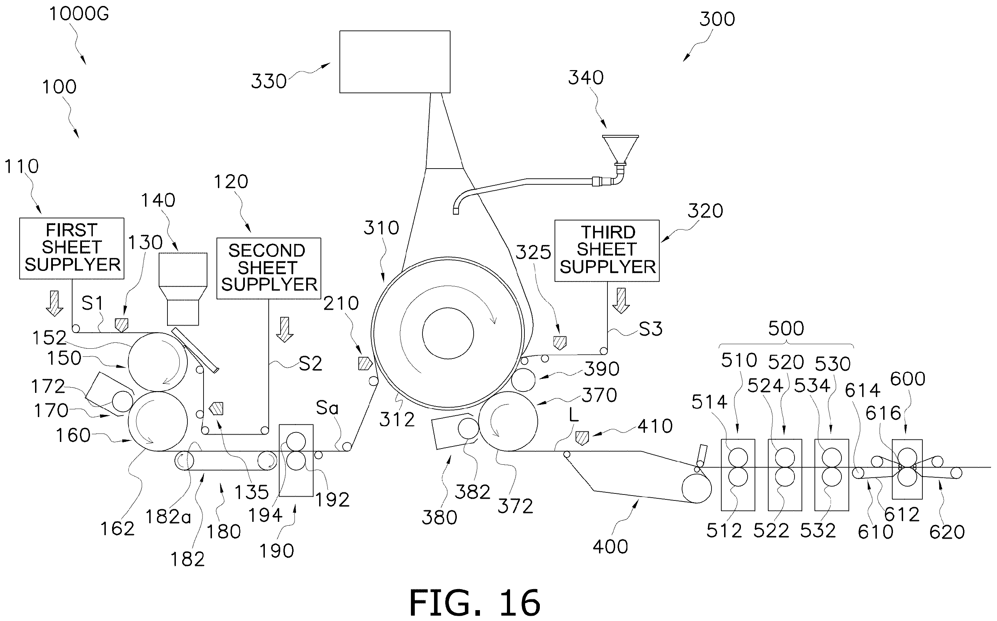

FIG. 16 is a schematic process diagram of an apparatus for manufacturing an absorbent body according to a seventh embodiment of the present invention.

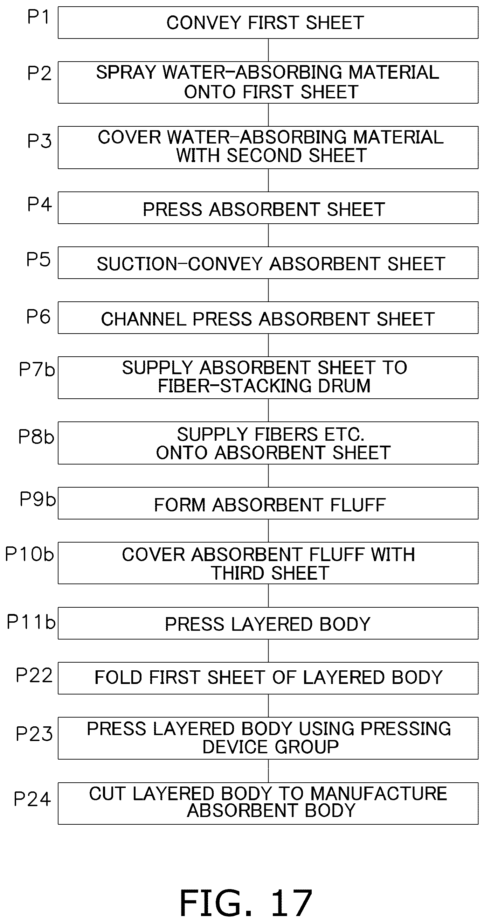

FIG. 17 is a schematic flowchart of a method for manufacturing an absorbent body according to the seventh embodiment of the present invention.

### DESCRIPTION OF EMBODIMENTS

Embodiments of an apparatus and method for manufacturing an absorbent body according to the present invention shall be described with reference to the drawings. The configuration of each embodiment described below may be combined with the configurations of other embodiments, as appropriate, as long as there are no particular contradictions.

The embodiments described below are merely examples of the present invention and are not intended to limit the scope of the present invention. A person skilled in the art would understand that various changes can be made to the following embodiments without departing from the spirit and scope of the present invention as set forth in the claims.

### (1) Absorbent body

An absorbent body 1 manufactured by the manufacturing apparatus and manufacturing method of the present invention shall be described with reference to FIGS. 1 and 2.

FIG. 1 is a schematic plan view of the absorbent body 1. The plan view of the absorbent body 1 in FIG. 1 is one example of the absorbent body 1 as seen along a thickness direction of the absorbent body 1 (a direction in which a first core 2 and a second core 3, described hereinafter, are layered). FIG. 2A is a cross-sectional view, taken along line II-II in FIG. 1, of a case in which the absorbent body 1 of FIG. 1 includes three sheets S1-S3. FIG. 2B is a cross-sectional view, taken along line II-II in FIG. 1, of a case in which the absorbent body 1 of FIG. 1 includes four sheets S1-S4.

In FIGS. 1 and 2, for the sake of convenience of explanation, the absorbent body 1 manufactured using the manufacturing apparatus and manufacturing method of the present invention is depicted in a simplified form.

For example, FIGS. 1 and 2 do not limit the shape of the absorbent body 1 to be manufactured. FIGS. 1 and 2 are drawn on the assumption that the absorbent sheet and absorbent fluff described hereinafter, which are manufactured using the manufacturing apparatus and manufacturing method of the present invention, have approximately the same shape and size when viewed along a thickness direction. However, if the absorbent sheet and the fluff absorbent have different shapes and sizes when viewed along the thickness direction, the plan view and cross-sectional view of the absorbent body 1 may be different from those depicted in FIGS. 1 and 2.

For example, the absorbent sheet may have a smaller longitudinal-direction length than the absorbent fluff. In such instances, for example, areas lacking the absorbent sheet are formed at both ends of the absorbent body in the longitudinal direction.

In FIG. 1, the absorbent body 1 is depicted as having a rectangular shape in plan view, and one channel C extending in the longitudinal direction of the absorbent body 1 is formed in the center of the absorbent body 1. However, FIG. 1 does not limit the shape of the absorbent body 1 or the position, shape, number, etc., of the channels C in the absorbent body 1 (in other words, the shape, etc., of the absorbent sheet or absorbent fluff formed by the manufacturing apparatus and manufacturing process of the present disclosure).

FIGS. 1 and 2 are merely drawings for the purpose of explanation, and the length and thickness of each member in each drawing do not represent the ratio of the dimensions of the actual absorbent body 1.

The absorbent body 1 manufactured using the manufacturing apparatus and manufacturing method of the present invention is used, for example, in disposable diapers, sanitary napkins, etc., and is an article that absorbs body fluids such as urine and menstrual blood.

In the example of FIG. 2A, the absorbent body 1 mainly includes a first core 2, a second core 3, and a third sheet piece S3p. In the example of FIG. 2B, the absorbent body 1 mainly includes a first core 2, a second core 3, a third sheet piece S3p, and a fourth sheet piece S4p. The first core 2 forms a water-absorbing material absorbent layer of the absorbent body 1. The second core 3 forms a stacked fiber absorbent layer, containing at least fluff, of the absorbent body 1. In short, the absorbent body 1 has a hybrid structure having a water-absorbing material absorbent layer and a stacked fiber absorbent layer containing at least fluff.

The first core 2 is obtained by cutting an absorbent sheet Sa (described hereinafter) to a predetermined size. The second core 3 is obtained by cutting an absorbent fluff Fa (described hereinafter) to a predetermined size. The second core 3 is laid over the first core 2. For example, when the absorbent body 1 is used in a diaper or the like, the second core 3 is disposed closer to the skin (crotch) of the wearer than the first core 2. By placing not the water-absorbing material absorbent layer but the stacked fiber absorbent layer including fluff closer to the skin of the wearer, a comfortable diaper or the like can be realized.

The third sheet piece S3p covers the second core 3 on an opposite side to the side where the first core 2 is placed. The fourth sheet piece S4p is a sheet piece covering the second core 3 on the side where the first core 2 is placed.

Whether the absorbent body 1 includes the first core 2, the second core 3, and the third sheet piece S3p as in FIG. 2A, or includes the first core 2, the second core 3, the third sheet piece S3p, and the fourth sheet piece S4p as in FIG. 2B, depends on the manufacturing process (described hereinafter).

Less material is used in the absorbent body 1 having the configuration of FIG. 2A than in the absorbent body 1 having the configuration of FIG. 2B (because the fourth sheet piece S4p is not present), and therefore it is possible for the raw-material cost to be lower than with the absorbent body 1 having the configuration of FIG. 2B. By contrast, in the absorbent body 1 having the configuration of FIG. 2B, the second core 3 is sandwiched between the third sheet piece S3p and the fourth sheet piece S4p, so the second core 3 is more likely to maintain the desired shape, and the absorbent body 1 having the desired shape can be manufactured with high precision.

In the first core 2, a water-absorbing material such as a superabsorbent polymer (SAP) is disposed between the first sheet piece S1p and the second sheet piece S2p. The first core 2 may include a mixture of one or more types of water-absorbing materials, or may include a mixture of water-absorbing materials having different particle sizes. On the first core 2 are formed an area where the superabsorbent polymer is sprayed (the area where the superabsorbent polymer P is disposed in FIGS. 2A and 2B) and a channel area C1 where the superabsorbent polymer P is not sprayed (where the superabsorbent polymer P is not disposed). The channel area C1 is formed so as to extend in the longitudinal direction of the first core 2. The first sheet piece S1p of the first core 2 is adhered to an outer surface of the third sheet piece S3p in order to fix the first core 2 and the second core 3 together.

The second core 3 is an absorbent body formed by laminating water-absorbing fibers or a mixture of water-absorbing fibers and a superabsorbent polymer. On the second core 3 are formed a portion where fibers F (including a mixture of fibers and a superabsorbent polymer, the same applies hereinafter) are layered and a channel part C2 where there are no fibers F. The channel part C2 is formed so as to extend in the longitudinal direction of the second core 3.

In the absorbent body 1 manufactured using the manufacturing apparatus and manufacturing method of the present disclosure, the channel area C1 of the first core 2 and the channel part C2 of the second core 3 are disposed so as to at least partially overlap each other as seen in plan view (when the absorbent body 1 is viewed along the thickness direction). This is because, in the manufacturing apparatus and manufacturing method of the present disclosure, as shall be described hereinafter, the channel area A2 of the absorbent sheet Sa, which will ultimately become the first core 2, and a channel part Fa2 of the absorbent fluff Fa, which will ultimately become the second core 3, are disposed so as to overlap.

The channel area C1 of the first core 2 and the channel part C2 of the second core 3 form a channel C in the absorbent body 1. Since the superabsorbent polymer P and the fibers F are not disposed in the channel C, diffusion of body fluids through the channel C is likely to be promoted in the absorbent body 1. Therefore, the absorbent body 1 can cause the bodily fluids discharged into the absorbent body 1 to be absorbed through diffusion into the superabsorbent polymer P and the fibers F around the discharge location.

### (2) Apparatus and method for manufacturing absorbent body

Embodiments of the apparatus and method for manufacturing the absorbent body 1 shall be described with reference to the drawings.

### (2-1) First embodiment

An apparatus 1000A for manufacturing the absorbent body 1 of the first embodiment and an embodiment of the manufacturing method shall be described with reference to FIGS. 3-8.

FIG. 3A is a schematic process diagram of the apparatus 1000A for manufacturing the absorbent body 1 according to the first embodiment. FIG. 3B is a schematic process diagram of the apparatus 1000A for manufacturing the absorbent body 1 according to another example of the first embodiment. FIG. 4 is a flowchart of a method for manufacturing the absorbent body 1 according to the first embodiment. FIG. 5 is a view of an absorbent sheet Sa conveyed by a third suction conveyer 220 of an absorbent sheet formation unit 100 of the manufacturing apparatus 1000A, viewed along a thickness direction of the absorbent sheet Sa. FIG. 6 is a view of an absorbent fluff Fa formed by a fiber-stacking unit 300 of the manufacturing apparatus 1000A, viewed along a thickness direction of the absorbent fluff Fa. FIG. 7 is a diagram schematically illustrating a first conveying surface 152 of a first drum 150 of the absorbent sheet formation unit 100 of the manufacturing apparatus 1000A. FIG. 8 is a diagram schematically illustrating an outer peripheral surface 312 of a fiber-stacking drum 310 of the fiber-stacking unit 300 of the manufacturing apparatus 1000A.

### (2-1-1) Apparatus for manufacturing absorbent body

### (2-1-1-1 ) Summary

The apparatus 1 000A for manufacturing the absorbent body 1 of the first embodiment mainly includes the absorbent sheet formation unit 100, the fiber-stacking unit 300, a folder 400, a layered-body-press 500, and a cutter 600.

The absorbent sheet formation unit 100 forms an absorbent sheet Sa in which a water-absorbing material (a SAP or the like) is sprayed between a first sheet S1 and a second sheet S2 (an absorbent sheet Sa in which a water-absorbing material is disposed in a predetermined area between the first sheet S1 and the second sheet S2). The first sheet S1 and the second sheet S2 are cut by the cutter 600 to make a first sheet piece S1p and a second sheet piece S2p in the absorbent body 1. In addition, the absorbent sheet Sa is cut by the cutter 600 to make the first core 2 in the absorbent body 1.

The fiber-stacking unit 300 is disposed downstream of the absorbent sheet formation unit 100 in a conveyance direction of the absorbent sheet Sa. The fiber-stacking unit 300 is preferably disposed as near to the absorbent sheet formation unit 100 as possible in the conveyance direction of the absorbent sheet Sa. The fiber-stacking unit 300 stacks fibers to form an absorbent fluff Fa including at least fluff (cotton-like fibers), and stacks the absorbent fluff Fa on the absorbent sheet Sa, which is conveyed while being sucked. The wording "the absorbent sheet Sa is conveyed while being sucked" means that with the absorbent sheet Sa in contact with a conveying surface, the conveying surface is moved to convey the absorbent sheet Sa while air is drawn in through the conveying surface. The same applies to the meaning of the term "suction conveyance" used below. The absorbent fluff Fa is cut by the cutter 600 to make the second core 3 in the absorbent body 1.

The folder 400 is disposed downstream of the fiber-stacking unit 300 in the conveyance direction of the absorbent sheet Sa over which the absorbent fluff Fa is laid (the absorbent sheet Sa over which the absorbent fluff Fa is laid is referred to below as a layered body L). The folder 400 folds the first sheet S1 of the absorbent sheet Sa so that the first sheet S1 partially covers a third sheet piece S3 covering the absorbent fluff Fa, so as to envelope the layered absorbent body (a hybrid core including the absorbent sheet Sa and the absorbent fluff Fa) to maintain the shape thereof.

The layered-body-press 500 is disposed downstream of the folder 400 in the conveyance direction of the layered body L. The layered-body-press 500 includes a plurality of devices that press all or part of the layered body L in order to adjust the shape of the absorbent sheet Sa on which the absorbent fluff Fa is overlaid.

The cutter 600 is disposed downstream of the layered-body-press 500 in the conveyance direction of the layered body L. The cutter 600 cuts the layered body L to form the absorbent body 1.

### (2-1-1-2) Absorbent body (hybrid core composed of absorbent sheet and absorbent fluff)

The absorbent sheet Sa and the absorbent fluff Fa shall be described.

The absorbent sheet Sa is a sheet in which a water-absorbing material containing a SAP is sprayed between the first sheet S1 and the second sheet S2. As previously described, the absorbent sheet Sa is cut by the cutter 600 to make the first core 2 in the absorbent body 1. When the absorbent sheet Sa is viewed along the thickness direction of the absorbent sheet Sa (the direction in which the first sheet S1, the water-absorbing material, and the second sheet S2 are layered), a spraying area A1 where the water-absorbing material is sprayed and a channel area A2 where the water-absorbing material is not sprayed are formed between the first sheet S1 and the second sheet S2 of the absorbent sheet Sa (see FIG. 5). The spraying area A1 of the absorbent sheet Sa becomes an area where the superabsorbent polymer P is sprayed in the first core 2. The channel area A2 of the absorbent sheet Sa becomes a channel area C1 where the superabsorbent polymer P is not sprayed in the first core 2. In the absorbent body 1 according to the example of FIG. 1, the channel area C1 is surrounded by the spraying area, but this example is not provided by way of limitation. For example, the channel area may be formed so as to pass through the spraying area in the longitudinal direction of the absorbent body 1.

The absorbent fluff Fa is formed by stacking fibers (including a case where a mixture of fibers and the water-absorbing material; the same applies below). As previously described, the absorbent fluff Fa is cut by the cutter 600 to become the second core 3 in the absorbent body 1. When the absorbent fluff Fa is viewed in the thickness direction, the absorbent fluff Fa can be seen to have a stacked fiber part Fa1 on which the water-absorbing material is disposed (on which the water-absorbing material is stacked) and a channel part Fa2 on which the fibers are not disposed (see FIG. 6). In the example shown in FIG. 6, which is not provided by way of limitation, the channel part Fa2 is surrounded by the stacked fiber part Fa1. The stacked fiber part Fa1 of the absorbent fluff Fa becomes an area where the fibers F are disposed in the second core 3. The channel part Fa2 of the absorbent fluff Fa becomes the channel part C2 where the fibers F are not disposed in the second core 3.

When the absorbent sheet Sa and the absorbent fluff Fa are viewed along the thickness direction, the absorbent sheet Sa and the absorbent fluff Fa have approximately the same shape and size. However, the absorbent sheet Sa and the absorbent fluff Fa may have different shapes and sizes when viewed along the thickness direction. FIGS. 5 and 6 have been created for the convenience of explanation and do not limit the shapes of the absorbent sheet Sa and the absorbent fluff Fa, or the positions, shapes, numbers, etc., of the channel area A2 in the absorbent sheet Sa and the channel part Fa2 in the absorbent fluff Fa.

### (2-1-1-3) Absorbent sheet formation unit

The absorbent sheet formation unit 100 shown in FIG. 3A of the present embodiment has a first sheet supplier 110, a second sheet supplier 120, a first adhesive applier 130, a second adhesive applier 135, a water-absorbing material supplier 140, a first drum 150 serving as an example of the first suction conveyer, a second drum 160, a first absorbent-part-press 170, a second suction conveyer 180, a second channel-press 190, an auxiliary conveyer 200, a third adhesive applier 210, and a third suction conveyer 220.

The first sheet supplier 110 supplies the first sheet S1, which is unwound from a sheet roll (not shown), to the first drum 150, which serves as an example of the first suction conveyer. The first sheet S1 is a core wrap for enveloping the absorbent body, and is configured from, for example, a liquid-permeable nonwoven fabric sheet, tissue paper, or the like. Since the first sheet S1 will ultimately be folded by the folder 400 and laid over the third sheet piece S3, the first sheet S1 is wider than the second sheet S2.

The second sheet supplier 120 supplies the second sheet S2, which is unwound from a sheet roll (not shown), to the first drum 150. The second sheet S2 is, for example, a liquid-permeable nonwoven fabric sheet.

The first adhesive applier 130 applies an adhesive to a water-absorbing material adhesion surface of the first sheet S1 (a surface on which the water-absorbing material is to be adhered) conveyed to the first drum 150. The water-absorbing material adhesion surface of the first sheet S1 is a surface that does not face the outer peripheral surface (first conveying surface 152) of the first drum 150 when conveyed by the first drum 150.

As previously described, the spraying area A1 where the water-absorbing material is sprayed and the channel area A2 where the water-absorbing material is not sprayed are formed between the first sheet S1 and the second sheet S2 of the absorbent sheet Sa (see FIG. 5). Therefore, the water-absorbing material adhesion surface of the first sheet S1 includes a portion to which the water-absorbing material is adhered and a portion to which the water-absorbing material is not adhered. Therefore, it is an option that the first adhesive applier 130 apply the adhesive only to the portion of the first sheet S1 to which the water-absorbing material is adhered (referred to hereinafter as the water-absorbing material adhesion portion). Alternatively, the first adhesive applier 130 may apply the adhesive also to portions where water-absorbing material adhesion is not necessary (particularly the channel area A2). For example, the first adhesive applier 130 may apply the adhesive to all of the surface of the first sheet S1 that does not face the outer peripheral surface (first conveying surface 152) of the first drum 150. In order to bond the first sheet S1 and the second sheet S2 together, it is preferable to apply the adhesive to the first sheet S1 in a range area wider than the spraying area A1 and the channel area A2 in a width direction of the first sheet S1.

The second adhesive applier 135 applies the adhesive to a water-absorbing material adhesion surface (a surface to which the water-absorbing material is adhered) of the second sheet S2 conveyed to the first drum 150. The water-absorbing material adhesion surface of the second sheet S2 is a surface that faces the outer peripheral surface (first conveying surface 152) of the first drum 150 when conveyed by the first drum 150. As with the first adhesive applier 130, it is an option that the second adhesive applier 135 apply the adhesive to only the water-absorbing material adhesion portion of the second sheet S2, or also to portions where water-absorbing material adhesion is not necessary. In order to bond the first sheet S1 and the second sheet S2 together, it is preferable to apply the adhesive to the second sheet S2 in a range area wider than the range corresponding to the spraying area A1 and the channel area A2 in a width direction of the second sheet S2.

It is preferable that either one of the first adhesive applier 130 and the second adhesive applier 135 apply the adhesive to an area including the channel area A2. As a result, the first sheet S1 and the second sheet S2 are easily bonded in the channel area A2, and movement of the water-absorbing material in the width direction can be suppressed.

The first drum 150 is a cylindrical drum in which the first conveying surface 152 having a suction function is provided on the outer peripheral surface. The first drum 150 is connected to a vacuum pump or another apparatus (not shown) via a pipe. When the vacuum pump is operated and air is drawn into the first drum 150 through suction holes (not shown) provided in the first conveying surface 152, the suction function of the first conveying surface 152 is exhibited.

The first sheet S1 is supplied to the first conveying surface 152 of the first drum 150 along a circumferential direction of the first drum 150. The first drum 150 rotates as shown by the arrow in FIG. 3 and conveys the first sheet S1 while the first sheet S1 supplied by the first sheet supplier 110 is suctioned by the first conveying surface 152. That is, the first sheet S1 is wrapped around the first drum 150, and tension thus applied can suppress movement of the first sheet S1 due to vibrations or the like during conveyance.

The water-absorbing material supplier 140 sprays the water-absorbing material (superabsorbent polymer P) on the surface of the first sheet S1 to which the adhesive is applied by the first adhesive applier 130, the first sheet S1 being conveyed by the first drum 150. The first sheet S1 is an air-permeable sheet, and the water-absorbing material is pulled to an area where suction holes are provided in the first conveying surface 152 and is held in place by the adhesive applied by the first adhesive applier 130.

As previously described, the spraying area A1 where the water-absorbing material is disposed and the channel area A2 where the water-absorbing material is not disposed are formed on the absorbent sheet Sa (between the first sheet S1 and the second sheet S2 of the absorbent sheet Sa). Therefore, the suction holes of the first conveying surface 152 are disposed only in the area of the first conveying surface 152 that comes into contact with a back side of the water-absorbing material adhesion portion of the first sheet S1, as shown by the hatching in FIG. 7. Inside the area shown by hatching in FIG. 7, the rectangular area surrounded by dotted lines not marked with hatching (where suction holes are not disposed) is the portion of the first conveying surface 152 that faces the portion of the first sheet S1 that becomes the channel area A2 upon becoming part of the absorbent sheet Sa.

For example, in cases such as when the first adhesive applier 130 applies the adhesive only to the water-absorbing material adhesion portion of the first sheet S1, suction holes may be formed in the entire range of the first conveying surface 152 (the area corresponding to the width of the first sheet S1) that comes into contact with the first sheet S1 (the area between the double-dash lines in FIG. 7). From the viewpoint of precisely forming a water-absorbing material accumulation pattern, it is preferable to provide suction holes only in the portions corresponding to the water-absorbing material spraying area A1, as in the present embodiment.

After the first sheet S1 passes through the position where the water-absorbing material is sprayed by the water-absorbing material supplier 140 (immediately downstream of the position where the water-absorbing material is sprayed in a direction along which the first drum 150 rotates), the second sheet S2, to which the adhesive is applied by the second adhesive applier 135 on the surface facing the first conveying surface 152 of the first drum 150, is supplied along the circumferential direction of the first drum 150 to the first conveying surface 152 of the first drum 150 conveying the first sheet S1. Supplying the second sheet S2 early allows the water-absorbing material to be sandwiched between the first sheet S1 and the second sheet S2 at an early timing, and can prevent the accumulation pattern of the water-absorbing material from being disrupted by vibrations or the like during conveyance. When the second sheet S2 is supplied to the first drum 150 conveying the first sheet S1, the first drum 150 rotates as shown by the arrow in FIG. 3 while the first sheet S1 and the second sheet S2, with the water-absorbing material sandwiched therebetween, are suctioned to the first conveying surface 152, thus conveying the first sheet S1 and the second sheet S2 with the water-absorbing material sandwiched therebetween (in other words, the absorbent sheet Sa).

Although not shown in the drawings, a press (temporary press) similar to the first absorbent-part-press 170 described below may be disposed adjacent to the first drum 150 and immediately downstream of the location where the second sheet S2 is supplied to the first drum 150. The temporary press may press the spraying area A1 between the first sheet S1 and the second sheet S2 of the absorbent sheet Sa conveyed between the temporary press and the first drum 150. By providing such a temporary press and pressing the spraying area A1 between the first sheet S1 and the second sheet S2 immediately after the water-absorbing material has been supplied onto the first sheet S1, it is easy to dispose the water-absorbing material evenly (at roughly the same thickness) in the spraying area A1 of the absorbent sheet Sa.

The temporary press may be the same as a first channel-press 190a described below. In this case, the first sheet S1 and the second sheet S2 can be joined in the channel area A2, which makes it possible to suppress the movement of the water-absorbing material in the width direction of the absorbent sheet Sa, which is perpendicular to the conveyance direction, and to form the channel (channel area A2) more precisely.

In order to place a predetermined amount of water-absorbing material between the first sheet S1 and the second sheet S2, it is preferable that the aforementioned area of the first conveying surface 152 that contacts the back side of the water-absorbing material adhesion portion of the first sheet S1 is recessed further inward in a radial direction of the first drum 150 than other parts of the first conveying surface 152 of the first drum 150.

The second drum 160 is disposed downstream of the first drum 150 in the conveyance direction of the absorbent sheet Sa (a sheet in which a water-absorbing material is disposed between the first sheet S1 and the second sheet S2) and adjacent to the first drum 150. The absorbent sheet Sa is delivered from the first drum 150 to the second drum 160, and the absorbent sheet Sa is wrapped around the second drum 160.

There are no limitations as to the second drum 160; an outer peripheral surface 162 may have a suction function, as with the first drum 150. If the outer peripheral surface 162 of the second drum 160 has a suction function, when the absorbent sheet Sa is conveyed while in contact with the outer peripheral surface 162 of the second drum 160, movement of the water-absorbing material between the first sheet S1 and the second sheet S2 is likely to be suppressed.

The second drum 160 is disposed directly below the first drum 150 and in contact with the first drum. The second drum 160 rotates in the opposite direction of the first drum 150. With this configuration, the first sheet S1 (or the absorbent sheet Sa) is conveyed in a state of having been wrapped around half a circumference (or approximately half a circumference) of the first drum 150 and half a circumference of the second drum 160. In other words, the absorbent sheet Sa is wrapped around and conveyed by the first drum 150 and the second drum 160 so as to form an approximately reversed S-shape. As a result, tension can be applied to the absorbent sheet Sa, so that the absorbent sheet Sa is conveyed while having been spread out in the conveyance direction or in a direction orthogonal to the conveyance direction, and movement of the SAP sandwiched between the first sheet S1 and the second sheet S2 can be effectively suppressed.

The first absorbent-part-press 170 is one example of a press. The first absorbent-part-press 170 is disposed adjacent to the second drum 160, and is a device that presses the spraying area A1 between the first sheet S1 and the second sheet S2 of the absorbent sheet Sa conveyed between the first absorbent-part-press 170 and the second drum 160 (the absorbent sheet Sa wrapped around the second drum 160). In other words, the first absorbent-part-press 170 is a device that presses the portion where the SAP is disposed, which contributes to the absorption of the absorbent sheet Sa. Thus, due to the absorbent sheet formation unit 100 having the first absorbent-part-press 170 and the first absorbent-part-press 170 pressing the spraying area A1 of the absorbent sheet Sa, the water-absorbing material can be disposed evenly (at approximately uniform thickness) on the spraying area A1 of the absorbent sheet Sa.

Specifically, the first absorbent-part-press 170 has a flat roll (a roll having no bumps or recesses in the outer peripheral surface in the radial direction) 172 serving as an example of a first pressing member. The flat roll 172 is disposed such that a gap of a predetermined size is formed between the flat roll 172 and the second drum 160. The size of this gap can be appropriately set in accordance with the desired degree of pressing of the absorbent sheet Sa. The first absorbent-part-press 170 pushes the flat roll 172, via the absorbent sheet Sa, against the second drum 160, which is also a flat roll and which functions as part of the first absorbent-part-press 170, thereby primarily pressing the spraying area A1 of the absorbent sheet Sa. The first absorbent-part-press 170 sandwiches the absorbent sheet Sa between the flat roll 172 and the second drum 160, which is also a flat roll, and can therefore be called an entire press that presses the entire absorbent sheet Sa.

When the first absorbent-part-press 170 presses the absorbent sheet Sa, it is preferable that at least one of the flat roll 172 and the second drum 160 has a function for heating the absorbent sheet Sa (has a heater for heating the absorbent sheet Sa). The first absorbent-part-press 170 heats the absorbent sheet Sa to a temperature equal to or higher than the temperature at which the adhesive appliers 130, 135 apply the adhesives. In the first absorbent-part-press 170, at least one of the flat roll 172 and the second drum 160 has a heating function, and heating the adhesive applied to the first sheet S1 and the second sheet S2 increases the permeability of the adhesive, which makes it easier to stabilize the position of the water-absorbing material between the first sheet S1 and the second sheet S2 of the absorbent sheet Sa.

As shown in FIG. 3, the auxiliary conveyer 200 may be disposed adjacent to the second drum 160. The auxiliary conveyer 200 is disposed, for example, downstream of the location pressed by the first absorbent-part-press 170 in the conveyance direction of the absorbent sheet Sa. The auxiliary conveyer 200 includes a conveyor belt 202 that is wound around a roller and that extends partially along the circumferential direction of the second drum 160. The conveyor belt 202 of the auxiliary conveyer 200 and the outer peripheral surface 162 of the second drum 160 sandwich the absorbent sheet Sa conveyed along the outer peripheral surface 162 of the second drum 160. The presence of the auxiliary conveyer 200 makes it easier to suppress position shifting of the water-absorbing material in the absorbent sheet Sa.

From the perspective of suppressing the movement of the conveyed water-absorbing material, the first channel-press 190a, which is an example of a press, may be provided instead of the auxiliary conveyer 200, as shown in FIG. 3B. The first channel-press 190a includes a pattern roll 192a, serving as an example of a second pressing member, having protrusions (not shown) protruding radially outward from a portion of an outer peripheral surface of the pattern roll. The first channel-press 190a presses the channel area A2 between the first sheet S1 and the second sheet S2 of the absorbent sheet Sa conveyed between the pattern roll 192a and the second drum 160 (the absorbent sheet Sa wrapped around the second drum 160). Specifically, the protrusions of the pattern roll 192a are designed so as to come into contact with the channel area A2 of the absorbent sheet Sa when the absorbent sheet Sa passes between the pattern roll and the second drum 160, and the protrusions of the pattern roll 192a and the outer peripheral surface of the second drum 160 press the channel area A2 of the absorbent sheet Sa therebetween.

When the first channel-press 190a presses the channel area A2 of the absorbent sheet Sa, it is preferable that at least one of the pattern roll 192a and the second drum 160 has a function for heating the absorbent sheet Sa (includes a heater for heating the absorbent sheet Sa), similar to the first absorbent-part-press 170 previously described. When the first channel-press 190a is provided with a heating function, it is preferable that the second drum 160 (flat roll) functioning as part of the first channel-press 190a has the heating function.

From the viewpoint of forming the channel (channel area A2 of the absorbent sheet Sa) neatly, it is preferable that the first channel-press 190a be provided upstream of the first absorbent-part-press 170 in the conveyance direction of the absorbent sheet Sa, as shown in FIG 3B.

In another embodiment, it is an option to dispose only the first channel-press 190a out of the first absorbent-part-press 170 and the first channel-press 190a in a position adjacent to the second drum 160, and to omit the first absorbent-part-press 170.

As described in the present embodiment, the first absorbent-part-press 170 and/or the first channel-press 190a are/is disposed so as to press against the peripheral surface of the second drum 160. That is, the first absorbent-part-press 170 and/or the first channel-press 190a press(es) against the absorbent sheet Sa wrapped around the first drum 150 and the second drum 160. This allows the absorbent sheet Sa to be in a state in which the absorbent sheet Sa has been wrapped around the first drum 150 and the second drum 160 and movement of the water-absorbing material is suppressed, and after the pressing process, it is possible to form an accumulated pattern of the water-absorbing material that is less susceptible to the influence of vibrations and the like that occur when the absorbent sheet Sa is conveyed downstream.

The second suction conveyer 180 is disposed between the second drum 160 and the second channel-press 190 in the conveyance direction of the absorbent sheet Sa. The second suction conveyer 180 is a conveyor that has a suction function. The second suction conveyer 180 includes a conveyor belt 182 wound around a roller. A horizontally extending surface (second conveying surface 182a) of the conveyor belt 182 is provided with suction holes (not shown), and air is drawn in through the suction holes, thereby suctioning the absorbent sheet Sa conveyed from the second drum 160 onto the second conveying surface 182a. As far as the conveyor belt 182 can be made air-permeable, the conveyor belt 182 may be provided with elongated openings, slits, or the like instead of suction holes. The second conveying surface 182a of the conveyor belt 182 preferably extends from the second drum 160 to the second channel-press 190. In other words, the conveyor belt 182 preferably extends from directly below the second drum 160 to a position adjacent to rolls 192, 194 (described hereinafter) of the second channel-press 190. In the second suction conveyer 180, while the absorbent sheet Sa is being suctioned onto the second conveying surface 182a, the roller around which the conveyor belt 182 is wound is rotated in the direction of the arrow in FIG. 3, thereby conveying the absorbent sheet Sa to the second channel-press 190.

The second channel-press 190 is one example of the press. The second channel-press 190 has a pair of rolls 192, 194. One of the pair of rolls 192, 194 is a pattern roll having protrusions (not shown) that protrude radially outward on part of an outer peripheral surface thereof. The other of the pair of rolls 192, 194 is a flat roll having a smooth outer peripheral surface (a roll having no bumps or recesses on the outer peripheral surface in the radial direction). The second channel-press 190 presses the channel area A2 between the first sheet S1 and the second sheet S2 of the absorbent sheet Sa conveyed between the roll 192 and the roll 194. Specifically, the protrusions of the pattern roll, which is one of the pair of rolls 192, 194, are designed to come into contact with the channel area A2 of the absorbent sheet Sa when the roll 192 and the roll 194 rotate and the absorbent sheet Sa passes between the roll 192 and the roll 194, and the protrusions of the pattern roll and the outer peripheral surface of the flat roll press the channel area A2 of the absorbent sheet Sa therebetween.

Preferably, out of the pair of rolls 192, 194, the roll that comes into contact with the side of the absorbent sheet Sa opposite the surface on which the absorbent fluff Fa is laid is a pattern roll, and the roll that comes into contact with the surface of the absorbent sheet Sa on which the absorbent fluff Fa is laid is a flat roll. For example, in the example shown in FIGS. 3A and 3B, the roll 192 is a pattern roll and the roll 194 is a flat roll. Due to such a configuration, the surface of the absorbent sheet Sa on the side on which the absorbent fluff Fa is laid is easily kept flat, and it is easy to lay the absorbent fluff Fa on the absorbent sheet Sa without position shifting.

By positioning the second channel-press 190 and pressing the channel area A2 of the absorbent sheet Sa in this manner, it is possible to form the channel area A2, which has no water-absorbing material and contributes to the diffusion of bodily fluids, at the desired position of the absorbent sheet Sa with high precision.

When the second channel-press 190 presses the absorbent sheet Sa, it is preferable that at least one of the roll 192 and the roll 194 has a function for heating the absorbent sheet Sa (has a heater for heating the absorbent sheet Sa). The second channel-press 190 heats the absorbent sheet Sa to a temperature that, for example, is equal to or higher than the temperature at which the adhesive appliers 130, 135 apply the adhesive. Due to at least one of the roll 192 and the roll 194 having a heating function and the adhesive on the roll 192 and the roll 194 being heated, the permeability of the adhesive increases, which makes it easier to stabilize the position of the water-absorbing material between the first sheet S1 and the second sheet S2 of the absorbent sheet Sa. When the second channel-press 190 is provided with a heating function, it is preferable that out of the flat roll and the pattern roll serving as the rolls 192, 194, at least the flat roll has a heating function.

Preferably, both the previously described flat roll 172 of the first absorbent-part-press 170 and the roll 194 (flat roll) of the second channel-press have a heating function. In such instances, the absorbent sheet Sa can be heated from either side of the first sheet S1 and the second sheet S2 that sandwich the water-absorbing material, which makes it easier for the adhesive applied to both the first sheet S1 and the second sheet S2 to permeate, and the water-absorbing material sandwiched between the first sheet S1 and the second sheet S2 can be held in place more stably.

The second channel-press 190 is preferably disposed in a position close to the second drum. For example, the second channel-press 190 is disposed near the second drum 160 and below the water-absorbing material supplier 140. In other words, it is preferable that the conveying distance of the second suction conveyer 180 is as short as possible. This makes it possible to reduce vibrations during conveying until a channel is neatly formed by the second channel-press 190, and to maintain a favorable accumulation pattern of the water-absorbing material.

The third suction conveyer 220 is disposed between the second channel-press 190 and the fiber-stacking unit 300 in the conveyance direction of the absorbent sheet Sa. The third suction conveyer 220 is a conveyor having a suction function. The third suction conveyer 220 includes a conveyor belt 222 wound around a roller. Suction holes (not shown) are provided on a surface (third conveying surface 222a) of the conveyor belt 222, and air is drawn in through the suction holes, thereby suctioning the absorbent sheet Sa, which has been pressed by the second channel-press 190, onto the third conveying surface 222a. As far as the conveyor belt 222 is air-permeable, the conveyor belt 222 may be provided with elongated openings, slits, or the like instead of suction holes. In the third suction conveyer 220, the roller around which the conveyor belt 222 is wound is rotated in the direction of the arrow in FIG. 3 with the absorbent sheet Sa suctioned to the third conveying surface 222a, whereby the absorbent sheet Sa is conveyed to the position where the fiber-stacking unit 300 lays the absorbent fluff Fa over the absorbent sheet Sa (preferably downstream of the position where the fiber-stacking unit 300 lays the absorbent fluff Fa over the absorbent sheet Sa). The third suction conveyer 220 is preferably disposed in close proximity to the second channel-press 190 so that the third conveying surface 222a can suction the absorbent sheet Sa immediately after the absorbent sheet leaves the second channel-press 190. In addition, the third conveying surface 222a is preferably flat.

The third adhesive applier 210 applies an adhesive for bonding the absorbent sheet Sa and the absorbent fluff Fa together an upper surface of the absorbent sheet Sa (the surface on the side of the absorbent sheet Sa on which the absorbent fluff Fa is laid; in this case, the second sheet S2) conveyed by the third suction conveyer 220.

It is preferable that the third suction conveyer 220 be disposed outside the range in which the third adhesive applier 210 discharges the adhesive, and adjacent to the range in which the third adhesive applier 210 discharges the adhesive. Such an arrangement makes it possible for the third suction conveyer 220 to be disposed close to the second channel-press 190, and the adhesive to be prevented from being directly applied onto the belt.

### (2-1-1-4) Fiber-stacking unit

The fiber-stacking unit 300 has, for example, the fiber-stacking drum 310, a third sheet supplier 320, a fourth adhesive applier 325, a fiber supplier 330, a water-absorbing material supplier 340, a fourth sheet supplier 350, a fifth adhesive applier 355, and an auxiliary conveyer 360.

The third sheet supplier 320 supplies a third sheet S3 unwound from a sheet roll (not shown) to the fiber-stacking drum 310. The third sheet S3 is, for example, a sheet of liquid-permeable nonwoven fabric.

The fourth sheet supplier 350 supplies a fourth sheet S4 unwound from a sheet roll (not shown) to the fiber-stacking drum 310. The fourth sheet S4 is, for example, a sheet of liquid-permeable nonwoven fabric. When an absorbent body 1 such as is depicted in FIG. 2B is manufactured, a fourth sheet supplier 350 is provided to the fiber-stacking unit 300, but when an absorbent body 1 such as is depicted in FIG. 2A is manufactured, the fourth sheet supplier 350 is not required in the fiber-stacking unit 300.

The fiber-stacking drum 310 is a hollow, cylindrical rotating drum. The fiber-stacking drum 310 rotates around centrally extending axis of the cylindrical fiber-stacking drum 310 (see the arrow in FIG. 3). In the outer peripheral surface 312 of the fiber-stacking drum 310 is formed at least one recess 314 for accumulating fibers supplied by the fiber supplier 330 and water-absorbing material supplied by the water-absorbing material supplier 340 and forming the fibers and the water-absorbing material into an absorbent fluff Fa of a predetermined shape (see FIG. 8). The recess 314 in the outer peripheral surface 312 has a shape corresponding to the absorbent fluff Fa to be formed, so that the shape of the fibers (including the water-absorbing material) accumulated from the recess 314 will be the predetermined shape of the absorbent fluff Fa. A portion 316 in FIG. 8 is a portion corresponding to the channel part Fa2 of the absorbent fluff Fa, and is disposed farther outward in the radial direction of the fiber-stacking drum 310 than the recess 314 (so as to protrude in the radial direction in relation to the recess 314).

The structure of the fiber-stacking drum 310 and how the fiber-stacking drum 310 forms the absorbent fluff Fa shall be outlined.

A vacuum pump or another apparatus (not shown) is connected via a pipe to the fiber-stacking drum 310, and a negative pressure space connected to the vacuum pump is formed inside the fiber-stacking drum 310. The negative pressure section is disposed correspondingly with respect to a fiber-stacking section (the section where fibers and water-absorbing material are supplied to the fiber-stacking drum 310) shown in FIG. 3. In addition, a blower or another apparatus (not shown) is connected via a pipe to the fiber-stacking drum 310, and a positive pressure space connected to the vacuum pump is formed inside the fiber-stacking drum 310. The positive pressure space is disposed to correspond to the position where the fiber-stacking drum 310 delivers the absorbent fluff Fa to the third suction conveyer 220. The positions of the negative pressure space and the positive pressure space do not change when the fiber-stacking drum 310 rotates.

The negative pressure space and the positive pressure space communicate with a space outside the fiber-stacking drum 310 via an opening (not shown) formed in a bottom surface of the recess 314 of the outer peripheral surface 312 of the fiber-stacking drum 310, which rotates around the negative pressure space and the positive pressure space.

When the fiber-stacking drum 310 rotates and a certain portion of the outer peripheral surface 312 passes a position adjacent to the negative pressure space inside the fiber-stacking drum 310, between the outside of the fiber-stacking drum 310 and the negative pressure space, air is drawn from the outside to the inside of the outer peripheral surface 312 through an opening (not shown) formed in the hatched area in FIG. 8 at the bottom (inner surface in the radial direction) of the recess 314 on the outer peripheral surface 31. No opening for air to enter or exit is formed in the portion indicated by reference numeral 316 in FIG. 8 (the portion corresponding to the channel part Fa2).

When a certain portion of the outer peripheral surface 312 passes a position adjacent to the negative pressure space, fibers supplied by the fiber supplier 330 and water-absorbing material supplied by the water-absorbing material supplier 340 are supplied around that portion for a predetermined period of time. As described above, when the opening of the recess 314 passes a position adjacent to the negative pressure space, air is drawn into the negative pressure space from the space outside the fiber-stacking drum 310, fibers and water-absorbing material are taken into the recess 314 of the outer peripheral surface 312 along with the air, and the fibers are stacked. The opening formed at the bottom of the recess 314 of the outer peripheral surface 312 is designed to dimensions at which air is allowed to pass through but not allow fibers or water-absorbing material to pass through.

Even after passing through the spraying section indicated by the double-dash line arrow, a certain portion of the outer peripheral surface 312 remains adjacent to the negative pressure space until just before passing near the third conveying surface 222a of the conveyor belt 222 of the third suction conveyer 220. Therefore, the absorbent fluff Fa formed in the recess 314 is held within the recess 314 until just before being superimposed on the absorbent sheet Sa being conveyed by suction by the third suction conveyer 220.

When a certain portion of the outer peripheral surface 312 passes near the third conveying surface 222a of the conveyor belt 222 of the third suction conveyer 220, that portion is adjacent to the positive pressure space, the outside of the fiber-stacking drum 310 communicates with the positive pressure space via an opening (not shown) formed in the bottom of the recess 314 of the outer peripheral surface 312, and air is blown from the inside to the outside of the fiber-stacking drum 310. As a result, the absorbent fluff Fa that was held in the recess 314 is discharged from the recess 314 and is superimposed on the absorbent sheet Sa being conveyed by suction by the third suction conveyer 220.

When the fiber-stacking drum 310 rotates and a certain portion of the outer peripheral surface 312 passes a position adjacent to the negative pressure space inside the fiber-stacking drum 310, not only the fibers and water-absorbing material but also the supplied third sheet S3 are conveyed while being suctioned onto the outer peripheral surface 312 (pulled into the recess 314) by the suction function of the fiber-stacking drum 310. Furthermore, when the fiber-stacking drum 310 rotates and a certain portion of the outer peripheral surface 312 passes a position adjacent to the negative pressure space inside the fiber-stacking drum 310, the supplied fourth sheet S4 is also conveyed while being suctioned via the fibers and water-absorbing material stacked in the recess 314 of the outer peripheral surface 312 by the suction function of the fiber-stacking drum 310.

The third sheet S3 is supplied to the outer peripheral surface 312 of the fiber-stacking drum 310 (upstream of the spraying section shown in FIGS. 3A and 3B in the rotation direction of the fiber-stacking drum 310) before the fibers and the water-absorbing material are stacked in the recess 314 of the outer peripheral surface 312. Therefore, in the fiber-stacking drum 310, the fibers and the water-absorbing material are stacked on the third sheet S3. In addition, after the fibers and the water-absorbing material are stacked in the recess 314 of the outer peripheral surface 312 (downstream of the spraying section shown in FIGS. 3A and 3B in the rotation direction of the fiber-stacking drum 310), the fourth sheet S4 is supplied to the fiber-stacking drum 310 so as to cover the fibers and the water-absorbing material stacked on the third sheet S3 (so that the fibers and the water-absorbing material are sandwiched between the third sheet S3 and the fourth sheet S4). In other words, the fourth sheet S4 is supplied to the fiber-stacking drum 310 so as to cover the absorbent fluff Fa formed in the recess 314 of the outer peripheral surface 312 (so that the absorbent fluff Fa comes to be sandwiched between the third sheet S3 and the fourth sheet S4). As mentioned above, the fourth sheet S4 is not essential.

The fourth adhesive applier 325 applies adhesive to the surface of the third sheet S3 on which the fibers and water-absorbing material are stacked before the third sheet S3 is supplied to the fiber-stacking drum 310. The surface of the third sheet S3 on which the fibers and water-absorbing material are stacked is the surface that does not face the outer peripheral surface 312 of the fiber-stacking drum 310. It is also an option that the fourth adhesive applier 325 apply adhesive only to the portion where the stacked fiber part Fa1 is provided (the portion that will fit into the recess 314 of the third sheet S3), or apply adhesive to the portion where the stacked fiber part Fa1 is provided as well as to locations other than the portion where the stacked fiber part Fa1 is provided.

The fifth adhesive applier 355 applies adhesive to the surface of the fourth sheet S4 that comes into contact with the fibers and water-absorbing material before the fourth sheet S4 is supplied to the fiber-stacking drum 310. The surface of the fourth sheet S4 that comes into contact with the fibers and water-absorbing material is the surface that faces the outer peripheral surface 312 of the fiber-stacking drum 310. It is also an option that the fifth adhesive applier 355 apply adhesive only to the portion of the fourth sheet S4 that comes into contact with the stacked fiber part Fa1, or apply adhesive to the portion that comes into contact with the stacked fiber part Fal as well as to locations other than the portion that comes into contact with the stacked fiber part Fa1.

The fiber supplier 330 crushes a pulp sheet formed by agglutinating pulp fibers into sheet form, producing flocculated fibers (pulp fibers). The water-absorbing material supplier 340 supplies a water-absorbing material (superabsorbent resin P) to the fiber-stacking drum 310. The mixture of the fibers produced by the fiber supplier 330 and the water-absorbing material supplied from the water-absorbing material supplier 340 is supplied so as to be blown out to the fiber-stacking drum 310 (around the outer peripheral surface 312 of the fiber-stacking drum 310) in a spraying section shown in FIGS. 3A and 3B.

The water-absorbing material supplied by the water-absorbing material supplier 340 and the water-absorbing material supplied by the water-absorbing material supplier 140 may be supplied from the same supply source (for example, a tank in which the water-absorbing material is stored). The water-absorbing material supplier 340 does not need to be provided when the absorbent fluff Fa is formed only from fibers.

As shown in FIG. 3, the auxiliary conveyer 360 may be disposed in a position adjacent to the fiber-stacking drum 310. The auxiliary conveyer 360 is disposed, for example, downstream of the supply location of the fourth sheet S4 in the conveyance direction of the absorbent fluff Fa and as close as possible to the supply location of the fourth sheet S4. The auxiliary conveyer 360 includes a conveyor belt 362 that is wound around a roller and that extends partially along the circumferential direction of the fiber-stacking drum 310. The conveyor belt 362 of the auxiliary conveyer 360 and the outer peripheral surface 312 of the fiber-stacking drum 310 sandwich the absorbent fluff Fa conveyed along the outer peripheral surface 312 of the fiber-stacking drum 310. Position shifting of the absorbent fluff Fa is more likely to be suppressed due to the presence of the auxiliary conveyer 360.

The fiber-stacking drum 310 stacks the formed absorbent fluff Fa on the absorbent sheet Sa (on the surface of the absorbent sheet Sa on which adhesive has been applied by the third adhesive applier 210) conveyed by the third suction conveyer 220. The absorbent sheet Sa on which the absorbent fluff Fa is stacked shall be referred to as a layered body L hereinafter. At this time, the fiber-stacking unit 300 stacks the channel part Fa2 of the absorbent fluff Fa on the channel area A2 of the absorbent sheet Sa. The fiber-stacking unit 300 stacking the channel part Fa2 of the absorbent fluff Fa with the channel area A2 of the absorbent sheet Sa includes a case in which the channel part Fa2 of the absorbent fluff Fa and the channel area A2 of the absorbent sheet Sa are completely overlapped. In addition, for example, when one of the channel part Fa2 of the absorbent fluff Fa and the channel area A2 of the absorbent sheet Sa is smaller than the other, this includes a case in which the smaller of the channel part Fa2 of the absorbent fluff Fa and the channel area A2 of the absorbent sheet Sa is encompassed by the larger of the channel part Fa2 of the absorbent fluff Fa and the channel area A2 of the absorbent sheet Sa. Furthermore, "the fiber-stacking unit 300 overlaps the channel part Fa2 of the absorbent fluff Fa with the channel area A2 of the absorbent sheet Sa" includes a case in which only a part of the channel part Fa2 of the absorbent fluff Fa is overlapped with the channel area A2 of the absorbent sheet Sa. By overlapping the absorbent sheet Sa and the absorbent fluff Fa so that the channel area A2 of the absorbent sheet Sa overlaps with the channel part Fa2 of the absorbent fluff Fa, the diffusion of bodily fluids through the channel C (corresponding to the channel area A2 of the absorbent sheet Sa and the channel part Fa2 of the absorbent fluff Fa) can be facilitated in the ultimately absorbent body 1, and the absorption performance of the absorbent body 1 can also be improved.

When the fiber-stacking drum 310 stacks the absorbent fluff Fa on the absorbent sheet Sa conveyed by the third suction conveyer 220 (places the absorbent fluff Fa on the absorbent sheet Sa), the third sheet S3 of the absorbent body 1 comes to be disposed on the side that does not face the absorbent sheet Sa, as shown in FIG. 3. In other words, the third sheet S3 supplied by the third sheet supplier 320 covers the absorbent fluff Fa disposed on top of the absorbent sheet Sa. In yet other words, the third sheet S3 is disposed on the opposite side of the absorbent sheet Sa, with the absorbent fluff Fa sandwiched therebetween.

### (2-1-1-5) Folder

The folder 400 is disposed downstream of the fiber-stacking unit 300 in the conveyance direction of the layered body L. In order to fix the first sheet S1 and the third sheet S3 together (so that when the absorbent body 1 is formed, the first sheet piece S1p comes to be fixed to the third sheet piece S3p), the folder 400 folds the first sheet S1 inward on both sides orthogonal to the conveyance direction of the layered body L so that the first sheet S1 of the absorbent sheet Sa at least partially covers the third sheet S3 covering the absorbent fluff Fa.

Before the first sheet S1 is folded inward by the folder 400, an adhesive for bonding the first sheet S1 and the third sheet S3 together is applied by a sixth adhesive applier 410 to the water-absorbing material adhesion surface of the first sheet S1 that is not covered by the second sheet S2, or to the surface of the third sheet S3 that does not face the absorbent fluff Fa.

Though not shown in FIG. 3, in the conveyance direction of the layered body L, between the folder 400 and the location where the absorbent fluff Fa is layered on the absorbent sheet Sa, a device that presses the overlapping portion of the stacked fiber part Fa1 of the absorbent fluff Fa and the spraying area A1 of the absorbent sheet Sa (e.g., a device that presses the absorbent fluff Fa and the absorbent sheet Sa between a pair of flat rollers) may be provided in order to adjust the shape of the layered body L. If the entire absorbent sheet Sa and absorbent fluff Fa are sandwiched by a pair of flat rollers in such a press, the press can be called an entire press that presses the entire layered body L of the absorbent sheet Sa and the absorbent fluff Fa.

### (2-1-1-6) Layered-body-press

The layered-body-press 500 includes a plurality of devices that press all or part of the layered body L in order to adjust the shape of the layered body L (in other words, the absorbent sheet Sa over which the absorbent fluff Fa is laid).

For example, the layered-body-press 500 includes a second absorbent-part-press 510, a third channel-press 520, and an edge-press 530. The second absorbent-part-press 510, the third channel-press 520, and the edge-press 530 are disposed in this order in the conveyance direction of the layered body L. However, the order in which the second absorbent-part-press 510, the third channel-press 520, and the edge-press 530 are disposed in the conveyance direction of the layered body L may be changed as appropriate.

The layered-body-press 500 preferably includes all of the second absorbent-part-press 510, the third channel-press 520, and the edge-press 530, but may include one or two of these. The layered-body-press 500 may also include two or more of at least one of the second absorbent-part-press 510, the third channel-press 520, and the edge-press 530. The layered-body-press 500 may also include press other than the second absorbent-part-press 510, the third channel-press 520, and the edge-press 530.

The second absorbent-part-press 510 has a pair of flat rolls 512, 514 and presses at least the overlapping portion between the stacked fiber part Fa1 of the absorbent fluff Fa and the spraying area A1 of the absorbent sheet Sa of the layered body L conveyed between the pair of flat rolls 512, 514. For example, the second absorbent-part-press 510 sandwiches the entire layered body L conveyed between the pair of flat rolls 512, 514. In this case, the second absorbent-part-press 510 can also be called an entire press that presses the entire layered body L of the absorbent sheet Sa and the absorbent fluff Fa. At least one of the flat rolls 512, 514 of the second absorbent-part-press 510 may be provided with a heater, and the second absorbent-part-press 510 may have a heating function for heating the layered body L. The second absorbent-part-press 510 heats the layered body L to, for example, a temperature equal to or higher than the temperature at which various the adhesive appliers apply the adhesive.

The third channel-press 520 has a pair of rolls 522, 524. In the third channel-press 520, as with the second channel-press 190, one of the pair of rolls 522, 524 is a flat roll, and the other of the pair of rolls 522, 524 is a pattern roll having protrusions (not shown) that protrude radially outward from a portion of an outer peripheral surface of the pattern roll. The third channel-press 520 presses the channel region A2 of the absorbent sheet Sa and the stacked fiber part Fa1 of the absorbent fluff Fa conveyed between the roll 522 and the roll 524. Specifically, the protrusions of the pattern roll, which is one of the pair of rolls 522, 524, are designed so that when the rolls 522, 524 rotate and the layered body L passes between the rolls 522, 524, the channel region A2 of the absorbent sheet Sa and the stacked fiber part Fa1 of the absorbent fluff Fa are sandwiched between the flat roll and the protrusions of the pattern roll. In this embodiment, the protrusions that become the channel pattern are formed on the roll 524 positioned on the absorbent fluff Fa side. As a result, a channel that makes diffusion of liquid more likely can be formed.

At least one of the rolls 522, 524 of the third channel-press 520 may be provided with a heater, and the third channel-press 520 may have a heating function for heating the layered body L. The third channel-press 520 heats the layered body L to a temperature equal to or higher than the temperature at which the various adhesive appliers apply the adhesive.

The edge-press 530 has a pair of rolls 532, 534. In the edge-press 530, one of the pair of rolls 532, 534 is a flat roll, and the other of the pair of rolls 532, 534 is a pattern roll having protrusions (not shown) that protrude radially outward on part of an outer peripheral surface of the pattern roll. The edge-press 530 presses the layered body L conveyed between the roll 532 and the roll 534 in a direction orthogonal to the conveyance direction of the layered body L. Specifically, the edge-press 530 presses the individual absorbent body 1, which will be formed by cutting with the cutter 600 disposed downstream, at both ends of individual absorbent body 1 in the conveyance direction. Specifically, the protrusions of the pattern roll, which is one of the pair of rolls 532, 534, are designed so that when the rolls 532, 534 rotate and the layered body L passes between the rolls 532, 534, both longitudinal ends of the layered body L in the conveyance direction corresponding to the individual absorbent bodies 1 are sandwiched between the protrusions of the pattern roll and the flat roll. A heater may be provided on at least one of the rolls 532, 534 of the edge-press 530, and the edge-press 530 may have a heating function for heating the layered body L. The edge-press 530 heats the layered body L to, for example, a temperature equal to or higher than the temperature at which the various adhesive appliers apply the adhesive.

### (2-1-1-7) Cutter

The cutter 600 is a device (mat cutter device) that forms an absorbent body 1 by cutting the layered body L pressed by the press of the layered-body-press 500 at predetermined intervals in the conveyance direction along a direction orthogonal to the conveyance direction.

The layered body L pressed by the layered-body-press 500 is conveyed to the cutter 600 by a conveyer 610.

For example, the conveyer 610 has two conveyor belts 612 wound around rollers 614, 616, and sandwiches the layered body L between the pair of conveyor belts 612 to convey the layered body L to the cutter 600. The pair of conveyor belts 612 of the conveyer 610 are disposed one above the other. The roller 616 disposed near the cutter 600 has as small a diameter as possible so that the layered body L introduced into the cutter 600 is held by the conveyor belts 612 up to close proximity to the cutter 600. This allows the conveyor belts 612 to be installed inside a housing of the cutter 600, up to close proximity to a cutting main body part composed of a cutter or the like.

The conveyer 610 is not limited to one having a pair of conveyor belts 612, but may be a conveyer having a suction function, as with the second suction conveyer 180 and the third suction conveyer 220 described above.

Whether the conveyer 610 has a pair of conveyor belts 612 or is a conveyer having a suction function, it is preferable that the conveyer 610 holds the layered body L throughout the entire area between the layered-body-press 500 and the cutter 600 (by sandwiching the layered body L via the conveyor belts 612 or by suction-holding the layered body L).

The layered body L (in other words, the absorbent body 1) cut by the cutter 600 is conveyed by a conveyer 620. The conveyer 620 is disposed symmetrically to the conveyer 610 with respect to the cutter 600. The conveyer 620 can have the same structure as the conveyer 610 except that the conveyer 620 is disposed symmetrically to the conveyer 610 and the conveyance direction is the opposite, so a description of the conveyer 620 shall be omitted here.

### (2-1-1-8) Method for manufacturing absorbent body

A method for manufacturing the absorbent body 1 shall be described with reference to the flowchart in FIG. 4. Details of each process have been described in the description of the manufacturing apparatus 1000A, so here the flow of manufacturing the absorbent body 1 shall be described. In the following description and FIG. 4, descriptions and illustrations of detailed steps shall be omitted.

First, a method for manufacturing the absorbent sheet Sa shall be described. In the process of manufacturing the absorbent sheet Sa, the first sheet S1, to which adhesive has been applied by the first adhesive applier 130, is conveyed by the first sheet supplier 110 and supplied to the first drum 150 (process P1). The water-absorbing material supplier 140 sprays water-absorbing material onto the first sheet S1 wrapped around the first drum 150 (process P2). Next, the second sheet S2, to which adhesive has been applied by the second adhesive applier 135, is supplied to the first drum 150, forming an absorbent sheet Sa in which the water-absorbing material is covered by the second sheet S2 (the water-absorbing material is sandwiched between the first sheet S1 and the second sheet S2) (process P3). After being delivered from the first drum 150 to the second drum 160, the absorbent sheet Sa is pressed by the first absorbent-part-press 170 and/or the first channel-press 190a (process P4). The pressed absorbent sheet Sa is delivered to the second suction conveyer 180, is conveyed by suction to the second channel-press 190 (process P4), and pressed by the second channel-press 190 (process P6). The adhesive is applied to the upper surface of the absorbent sheet Sa pressed by the second channel-press 190, and the absorbent sheet Sa is delivered to the third suction conveyer 220 and conveyed to a location where the absorbent fluff Fa is laid over the absorbent sheet Sa by the fiber-stacking unit 300 (process P7).

Next, the method for manufacturing the absorbent fluff Fa shall be described. In the process of manufacturing the absorbent fluff Fa, to which adhesive has been applied by the fourth adhesive applier 325, the third sheet S3 is supplied to the stacking drum 310 by the third sheet supplier 320 (process P11). The fiber-stacking drum 310 then draws air through an opening provided in the outer peripheral surface 312 through the third sheet S3. At this time, fibers are supplied around the outer peripheral surface 312 of the stacking drum 310 by the fiber supplier 330, and the water-absorbing material is supplied by the water-absorbing material supplier 340 (process P12). Therefore, fibers (including the water-absorbing material) are stacked in the recess 314 on the outer peripheral surface 312 of the fiber-stacking drum 310, forming the absorbent fluff Fa (process P13). The absorbent fluff Fa on the third sheet S3 may be covered by the fourth sheet S4 to which adhesive has been applied by the fifth adhesive applier 355 (process P14).

Next, the fiber-stacking unit 300 stacks the formed absorbent fluff Fa on the absorbent sheet Sa conveyed by suction by the third suction conveyer 220 (process P21). At this time, the channel part Fa2 of the absorbent fluff Fa is at least partially overlapped with the corresponding channel area A2 of the absorbent sheet Sa. In a plan view, since the channel part Fa2 of the absorbent fluff Fa and the channel area A2 of the absorbent sheet Sa have substantially the same shape and dimensions, the channel part Fa2 of the absorbent fluff Fa is almost completely overlapped with the corresponding channel area A2 of the absorbent sheet Sa.

The absorbent sheet Sa (layered body L) over which the absorbent fluff Fa is laid is sent to the folder 400. The folder 400 folds the first sheet S1 of the layered body L so as to overlap the third sheet S3 (process P22). The layered body L, from which the first sheet S1 has been folded by the folder 400, is pressed by the presss 510, 520, 530 of the layered-body-press 500 (process P23). After being pressed by the layered-body-press 500, the layered body L is sent by the conveyer 610 to the cutter 600 and cut into absorbent bodies 1 (process P24).

### (2-1-1-9) Modifications

Modifications of the manufacturing apparatus 1000A and the manufacturing method of the first embodiment shall be described.

### <Modification A>

In FIG. 3A of the above embodiment, the absorbent sheet formation unit 100 has two presses (the first absorbent-part-press 170 and the second channel-press 190), and in FIG. 3B of the above embodiment, the absorbent sheet formation unit 100 has three presses (the first absorbent-part-press 170, the first channel-press 190a, and the second channel-press 190). It is also an option for the absorbent sheet formation unit 100 to have only one of the first absorbent-part-press 170, the first channel-press 190a, and the second channel-press 190. Conversely, the absorbent sheet formation unit 100 may have presses (e.g., a press similar to the first absorbent-part-press 170 and a press similar to the second channel-press 190) other than the first absorbent-part-press 170, the first channel-press 190a, and the second channel-press 190 provided in appropriate locations.

### <Modification B>

In the configuration of FIG. 3B of the above embodiment, the second channel-press 190 may be omitted as in FIG. 9.

### <Modification C>

In the above embodiment, the first suction conveyer in the claims was described as the first drum 150, but the first suction conveyer may be a conveyer that has a conveyor belt having a suction function similar to that of the second suction conveyer 180 and the third suction conveyer 220.

In such instances, the first absorbent-part-press 170 may be, as with the above embodiment, a device that presses the absorbent sheet Sa between the flat roll 172 serving as a first pressing member and the second drum 160 disposed downstream of the first suction conveyer, or, as with the second channel-press 190, a device that uses two rolls to press the absorbent sheet Sa conveyed by a first suction conveyer having a conveyor belt.

### <Modification D>

The order in which the first absorbent-part-press 170, the first channel-press 190a, and the second channel-press 190 are disposed in the above embodiment may be changed as appropriate.

### <Modification E>

In the above embodiment, a temporary press provided to the first drum 150, the first channel-press 190a, and the second channel-press 190 may press not only the channel area A2 exemplified in the above embodiment, but also both ends of the spraying area A1 of the absorbent sheet Sa in the conveyance direction of the absorbent sheet Sa and/or both ends of the spraying area A1 of the absorbent sheet Sa in a direction orthogonal to the conveyance direction of the absorbent sheet Sa, so as to surround the SAP spraying area A1.

### <Modification F>

Instead of the channel-press and edge-press in the above embodiment, a heat sealing device, ultrasonic sealing device, or the like may be provided to join the sheets together.

### (2-2) Second embodiment

An embodiment of a device 1000B and method for manufacturing the absorbent body 1 of a second embodiment shall be described with reference to FIG. 10. FIG. 10 is a schematic process diagram of the device 1000B for manufacturing the absorbent body 1 according to the second embodiment.

The manufacturing device 1000B is, in many respects, similar to the manufacturing apparatus 1000A shown in FIG. 3A of the first embodiment. The differences between the manufacturing device 1000B and the manufacturing apparatus 1000A shall now be described and descriptions of similar features shall be omitted.

The main difference that the manufacturing device 1000B has with the manufacturing apparatus 1000A is that the fiber-stacking unit 300 has a transfer drum 370 and a second stacked-fiber-part-press 380, which the manufacturing apparatus 1000A does not have.

The transfer drum 370 is disposed downstream of the fiber-stacking drum 310 in the conveyance direction of the absorbent fluff Fa, receives the absorbent fluff Fa from the fiber-stacking drum 310, and conveys the absorbent fluff Fa to a position where the absorbent fluff Fa will be laid over on the absorbent sheet Sa.

The second stacked-fiber-part-press 380 is disposed adjacent to the transfer drum 370. The second stacked-fiber-part-press 380 has a flat roll 382 (see FIG. 10) serving as a pressing member, and, in a state in which the absorbent fluff Fa is disposed between the flat roll 382 and the transfer drum 370, which is a flat roll functioning as part of the second stacked-fiber-part-press 380, the flat roll 382 is pushed against an outer peripheral surface 372 of the transfer drum 370 via the absorbent fluff Fa, and the stacked fiber part Fa1 of the absorbent fluff Fa is pressed. Providing the second stacked-fiber-part-press 380 in this manner makes it easier to obtain an absorbent fluff Fa in a desired shape.

The absorbent fluff Fa pressed by the second stacked-fiber-part-press 380 is then superimposed on the absorbent sheet Sa by the third suction conveyer 220. The second stacked-fiber-part-press 380 may be configured as a channel-press that presses the channel part Fa2 of the absorbent fluff Fa.

The fiber-stacking unit 300 of the manufacturing device 1000B may have a first stacked-fiber-part-press 390 that the manufacturing apparatus 1000A does not have. The first stacked-fiber-part-press 390 is a device that presses the stacked fiber part Fa1 of the absorbent fluff Fa between a flat roll 392 and the fiber-stacking drum 310 in which the recess 314 is formed in the outer peripheral surface 312. Providing the first stacked-fiber-part-press 390 in this manner makes it easier to obtain the absorbent fluff Fa in a desired shape. In particular, providing the first stacked-fiber-part-press 390 and the second stacked-fiber-part-press 380 and pressing the absorbent fluff Fa in two stages makes it possible to stably convey the absorbent fluff Fa in a desired shape.

The flow of the manufacturing method of the second embodiment using the manufacturing device 1000B includes a pressing process performed by the first stacked-fiber-part-press 390 and a pressing process performed by the second stacked-fiber-part-press 380 after process P14 in FIG. 4 and before process P21 in FIG. 4. An illustration of a flowchart of the entire manufacturing method of the second embodiment is omitted.

Another difference between the manufacturing device 1000B and the manufacturing apparatus 1000A is that the auxiliary conveyer 200 of the absorbent sheet formation unit 100 and the auxiliary conveyer 360 of the fiber-stacking unit 300 are omitted in the example shown in FIG. 10. However, such an omission is not cited by way of limitation; the auxiliary conveyer 200 and the auxiliary conveyer 360 may be provided in the manufacturing device 1000B as well.

In addition, the positions at which constituent mechanisms are depicted differ between the fiber-stacking unit 300 of FIG. 10 and the fiber-stacking unit 300 of FIG. 3, but this is for depiction-related reasons. In all other respects, the fiber-stacking unit 300 of the manufacturing device 1000B and the fiber-stacking unit 300 of the manufacturing apparatus 1000A are identical.

### (2-3) Third embodiment

An embodiment of a device 1000C and method for manufacturing the absorbent body 1 of a third embodiment shall be described with reference to FIG. 11. FIG. 11 is a schematic process diagram of the device 1000C for manufacturing the absorbent body 1 according to the third embodiment.

The manufacturing device 1000C is, in many features, similar to the manufacturing apparatus 1000A shown in FIG. 3A of the first embodiment. The differences between the manufacturing device 1000C and the manufacturing apparatus 1000A shall be described and descriptions of similar features shall be omitted.

The main difference that the manufacturing device 1000C has with the manufacturing apparatus 1000A is that the fiber-stacking unit 300 does not have the fourth sheet supplier 350, the fifth adhesive applier 355, or the auxiliary conveyer 360, and the third sheet supplier 320 does not supply the third sheet S3 to the fiber-stacking drum 310.

In the manufacturing device 1000C, instead of stacking the fibers and water-absorbing material on the third sheet S3 disposed on the outer peripheral surface 312 of the fiber-stacking drum 310, the fibers and water-absorbing material are stacked directly (without any other member interposed) on the outer peripheral surface 312 of the fiber-stacking drum 310 to form the absorbent fluff Fa. The fiber-stacking drum 310 then lays the absorbent fluff Fa, of which one surface is not covered by the third sheet S3, over the absorbent sheet Sa. At this time, the fiber-stacking drum 310 forms the layered body L by laying the absorbent fluff Fa over the absorbent sheet Sa such that the channel part Fa2 of the absorbent fluff Fa is disposed over the channel area A2, as in the first embodiment. In the manufacturing device 1000C, before the first sheet S1 of the layered body L is folded by the folder 400, the third sheet supplier 320 supplies the third sheet S3 so as to cover the absorbent fluff Fa of the layered body L conveyed by the third suction conveyer 220 (or a conveyer other than the third suction conveyer 220). It is preferable that the third sheet supplier 320 supplies the third sheet S3 near the contact point between the fiber-stacking drum 310 and the third suction conveyer 220. In this case, the absorbent fluff Fa is immediately covered by the third sheet S3 after being laid over the absorbent sheet Sa, and the desired shape of the layered body L can be well maintained.

The fiber-stacking unit 300 of the manufacturing device 1000C may have a first stacked-fiber-part-press 390 that the manufacturing apparatus 1000A does not have. The first stacked-fiber-part-press 390 is a device that presses the stacked fiber part Fa1 of the absorbent fluff Fa between the flat roll 392 and the fiber-stacking drum 310 in which the recess 314 is formed in the outer peripheral surface 312. Providing the first stacked-fiber-part-press 390 in this manner makes it easier to obtain the absorbent fluff Fa in a desired shape.

Another difference between the manufacturing device 1000B and the manufacturing apparatus 1000A is that the auxiliary conveyer 200 of the absorbent sheet formation unit 100 is omitted in the example shown in FIG. 11. However, such an omission is not cited by way of limitation; the auxiliary conveyer 200 may be provided in the manufacturing device 1000B as well.

The flow of the manufacturing method of the third embodiment using the manufacturing device 1000C is as shown in, for example, FIG. 12. The flow of the manufacturing method of the third embodiment and the flow of the manufacturing method of the first embodiment differ mainly in terms of the processes relating to forming the absorbent fluff Fa.

The processes P1-P7 in FIG. 12 are similar to the processes P1-P7 in FIG. 4 of the first embodiment.

In forming the absorbent fluff Fa, the fibers are supplied by the fiber supplier 330 (without the third sheet S3 being supplied) and the water-absorbing material is supplied by the water-absorbing material supplier 340 around the fiber-stacking drum 310, which draws air in through suction holes provided on the outer peripheral surface 312 (process P11a). As a result, fibers (including the water-absorbing material) are stacked in the recess 314 of the outer peripheral surface 312 of the fiber-stacking drum 310, and the absorbent fluff Fa is formed (process P12a). The absorbent fluff Fa is then pressed by the first stacked-fiber-part-press 390 (process P13a). Process P13a for pressing the absorbent fluff Fa may be omitted. Having undergone the pressing process, the absorbent fluff Fa is laid over the absorbent sheet Sa to which the adhesive has been applied by the third adhesive applier 210 (process P21). Thereafter, the third sheet S3, to which the adhesive has been applied by the fourth adhesive applier 325 on the surface facing the absorbent fluff Fa, is laid over the absorbent fluff of the layered body L, which is the result of laying the absorbent fluff Fa on the absorbent sheet Sa (process P21a). The steps of processes P22-P24 are similar to the processes P22-P24 of the flowchart of the manufacturing method of the first embodiment (see FIG. 4).

### (2-4) Fourth embodiment

An embodiment of a device 1000D and method for manufacturing the absorbent body 1 of a fourth embodiment shall be described with reference to FIG. 13. FIG. 13 is a schematic process diagram of the device 1000D for manufacturing the absorbent body 1 according to the fourth embodiment.

The manufacturing device 1000D is obtained by further providing the transfer drum 370 and the second stacked-fiber-part-press 380 of the manufacturing device 1000B of the second embodiment to the manufacturing device 1000C of the third embodiment.

The transfer drum 370 and the second stacked-fiber-part-press 380 of the manufacturing device 1000D of the fourth embodiment differ from the transfer drum 370 and the second stacked-fiber-part-press 380 of the manufacturing device 1000B of the second embodiment in that one surface of the absorbent fluff Fa delivered to the transfer drum 370 and pressed by the second stacked-fiber-part-press 380 is not covered by the third sheet S3. In other respects, the transfer drum 370 and the second stacked-fiber-part-press 380 of the manufacturing device 1000D of the fourth embodiment are similar to the transfer drum 370 and the second stacked-fiber-part-press 380 of the manufacturing device 1000B of the second embodiment; therefore, a detailed description thereof, including the description of the flow of the manufacturing method, has been omitted.

### (2-5) Fifth embodiment

An embodiment of a device 1000E and method for manufacturing the absorbent body 1 of a fifth embodiment shall be described with reference to FIG. 14. FIG. 14 is a schematic process diagram of the device 1000E for manufacturing the absorbent body 1 according to the fifth embodiment.

The manufacturing device 1000E is, in many respects, similar to the manufacturing apparatus 1000A shown in FIG. 3A of the first embodiment. The differences between the manufacturing device 1000E and the manufacturing apparatus 1000A shall be described and descriptions of similar features shall be omitted.

The main difference that the manufacturing device 1000E has with the manufacturing apparatus 1000A is that the fiber-stacking unit 300 further has a gear stretcher 327 that subjects the third sheet S3 to gear stretching. Due to such a configuration, the third sheet S3 is more likely to deform, and the third sheet S3 therefore is more likely to fit into the recess 314 for fiber stacking formed in the outer peripheral surface 312 of the fiber-stacking drum 310, making it easier to manufacture an absorbent body 1 in a desired shape (an absorbent body 1 having an absorbent fluff Fa in a desired shape).

The flow of the manufacturing method of the fifth embodiment using the manufacturing device 1000E includes a gear stretching process for the third sheet S3, which is executed before the third sheet S3 is supplied to the fiber-stacking drum 310 in process P11 of FIG. 4. An illustration of an overall flowchart of the manufacturing method of the fifth embodiment is omitted.

### (2-6) Sixth embodiment

An embodiment of a device 1000F and method for manufacturing the absorbent body 1 of a sixth embodiment shall be described with reference to FIG. 15. FIG. 15 is a schematic process diagram of the device 1000E for manufacturing the absorbent body 1 according to the fifth embodiment.

The manufacturing device 1000F is, in many features, similar to the manufacturing device 1000B of the second embodiment. The differences between the manufacturing device 1000F and the manufacturing device 1000B shall be described and descriptions of similar features shall be omitted.

The main difference that the manufacturing device 1000F has with the manufacturing device 1000B is that the fiber-stacking unit 300 further has a gear stretcher 327 that subjects the third sheet S3 to gear stretching. Due to such a configuration, the third sheet S3 is more likely to deform, and the third sheet S3 therefore is more likely to fit into the recess 314 for fiber stacking formed in the outer peripheral surface 312 of the fiber-stacking drum 310, making it easier to manufacture an absorbent body 1 in a desired shape (an absorbent body 1 having an absorbent fluff Fa in a desired shape).

Since the manufacturing device 1000F of the sixth embodiment is merely a combination of manufacturing devices and manufacturing methods already described, a detailed description, of the manufacturing device 1000F shall be omitted.

### (2-7) Seventh embodiment

An embodiment of a device 1000G and method for manufacturing the absorbent body 1 of a seventh embodiment shall be described with reference to FIG. 16. FIG. 16 is a schematic process diagram of the device 1000G for manufacturing the absorbent body 1 according to the seventh embodiment.

The absorbent sheet formation unit 100 of the manufacturing device 1000G is similar to the absorbent sheet formation unit 100 of the manufacturing device 1000B aside from not having the third suction conveyer 220.

A major difference that the fiber-stacking unit 300 of the manufacturing device 1000G has with the fiber-stacking unit 300 of the manufacturing device 1000B of the second embodiment is that the absorbent sheet Sa bonded by the third adhesive applier 210 is supplied to the fiber-stacking drum 310, and the fibers and water-absorbing material are stacked on the absorbent sheet Sa. In short, in the fiber-stacking unit 300 of the manufacturing device 1000G, after formation of the absorbent fluff Fa is complete, the absorbent fluff Fa is not laid over the absorbent sheet Sa, but the absorbent fluff Fa is formed on the absorbent sheet Sa conveyed by suction by the fiber-stacking drum 310.

The fiber-stacking unit 300 of the manufacturing device 1000G has a transfer drum 370, a first stacked-fiber-part-press 390, and a second stacked-fiber-part-press 380, as does the fiber-stacking unit 300 of the manufacturing device 1000B of the second embodiment, but the role of each component is different from those of the second embodiment. The transfer drum 370 of the fiber-stacking unit 300 of the manufacturing device 1000G is not a drum that receives and conveys the absorbent fluff Fa from the fiber-stacking drum 310, but is a drum that receives and conveys the layered body L in which the absorbent fluff Fa is laid over the absorbent sheet Sa. In addition, the first stacked-fiber-part-press 390 and the second stacked-fiber-part-press 380 are not devices that press the stacked fiber part Fa1 of the absorbent fluff Fa (an individual absorbent fluff Fa), but are devices that press the spraying area A1 of the absorbent sheet Sa and the stacked fiber part Fa1 of the absorbent fluff Fa.

The fiber-stacking unit 300 of the manufacturing device 1000G also differs from the fiber-stacking unit 300 of the manufacturing device 1000B of the second embodiment by not having a fourth sheet supplier 250 or a fifth adhesive applier 335.

In other respects, the manufacturing device 1000G is similar to the manufacturing device 1000B of the second embodiment, and therefore a description thereof shall be omitted here.

The flow of the manufacturing method of the seventh embodiment using the manufacturing device 1000G is, for example, as shown in FIG. 17.

Processes P1-P7 in FIG. 17 are similar to processes P1-P6 in FIG. 4 of the first embodiment.

In the manufacturing method of the seventh embodiment, the absorbent sheet Sa is supplied to the fiber-stacking drum 310 (process P7b) after process P6. The fiber-stacking drum 310 then draws in air from the suction holes provided in the outer peripheral surface 312, through the absorbent sheet Sa. At this time, around the outer peripheral surface 312 of the fiber-stacking drum 310, fibers are supplied by the fiber supplier 330 and water-absorbing material is supplied by the water-absorbing material supplier 340 (process P8b). Therefore, fibers (including water-absorbing material) are stacked on the absorbent sheet Sa and the absorbent fluff Fa is formed on the absorbent sheet Sa in the recess 314 of the outer peripheral surface 312 of the fiber-stacking drum 310 (process P9b). The absorbent fluff Fa on the absorbent sheet Sa is then covered by the third sheet S3 in which adhesive has been applied by the fourth adhesive applier 325 to the surface on the side facing the absorbent fluff Fa (process P10b). The absorbent sheet Sa on which the absorbent fluff Fa is laid (the layered body L) is then pressed by the first stacked-fiber-part-press 390 and the second stacked-fiber-part-press 380 (process P11b), and then sent to the folder 400.

Processes P22-P24 in FIG. 17 are similar to processes P22-24 in FIG. 4, and descriptions thereof shall therefore be omitted.

### (3) Characteristics

(3-1)
The apparatuses 1000A-1000G for manufacturing the absorbent body 1 are apparatuses for manufacturing a hybrid structure absorbent body 1 having a water-absorbing material absorbent layer and a stacked fiber absorbent layer including at least fluff. The apparatuses 1000A-1000G for manufacturing the absorbent body 1 each include an absorbent sheet formation unit 100 and a fiber-stacking unit 300. The absorbent sheet formation unit 100 forms, between a first sheet S1 and a second sheet S2, an absorbent sheet Sa in which a water-absorbing material containing a SAP or the like is sprayed. The fiber-stacking unit 300 stacks fibers to form an absorbent fluff Fa including at least fluff, and stacks the absorbent fluff Fa on the absorbent sheet Sa, which is conveyed while being sucked. A spraying area A1 and a channel area A2 are formed between the first sheet S1 and the second sheet S2 of the absorbent sheet Sa. The water-absorbing material is sprayed in the spraying area A1. The water-absorbing material is not sprayed in the channel area A2. The absorbent fluff Fa has a stacked fiber part Fa1 and a channel part Fa2. The fibers are stacked in the stacked fiber part Fa1. The fibers are not stacked in the channel part Fa2. The absorbent sheet formation unit 100 has a first sheet supplier 110, a first drum 150, which is one example of a first suction conveyer, a water-absorbing material supplier 140, a second sheet supplier 120, and a press. The first sheet supplier 110 supplies the first sheet. The first drum 150 has a first conveying surface 152 having a suction function, and conveys the first sheet S1 while suctioning the first sheet S1 on the first conveying surface 152. The water-absorbing material supplier 140 supplies the water-absorbing material onto the first sheet S1 conveyed by the first drum 150. The second sheet supplier 120 supplies the second sheet S2 and covers the water-absorbing material on the first sheet S1 with the second sheet S2 to form the absorbent sheet Sa. The press presses the conveyed absorbent sheet Sa. In the above embodiments, the press includes at least one of a first absorbent-part-press 170, a first channel-press 190a, and a second channel-press 190. The fiber-stacking unit 300 has a fiber supplier 330, a fiber-stacking drum 310, and a third sheet supplier 320. The fiber supplier 330 supplies the fibers. The fiber-stacking drum 310 has a suction function, and stacks the fibers supplied by the fiber supplier 330 to form the absorbent fluff Fa. The third sheet supplier 320 supplies a third sheet S3 that is disposed on the side opposite the absorbent sheet Sa with the absorbent fluff Fa sandwiched therebetween. When stacking the absorbent fluff Fa on the absorbent sheet Sa, the fiber-stacking unit 300 lays the channel part Fa2 of the absorbent fluff Fa over the channel area A2 of the absorbent sheet Sa.

In the apparatuses 1000A-1000G for manufacturing the absorbent body 1, the absorbent sheet Sa, in which the position of the water-absorbing material is likely to shift, is conveyed by suction, and the absorbent fluff Fa is superimposed on the absorbent sheet Sa. Therefore, the apparatuses 1000A-1000G for manufacturing the absorbent body 1 can suppress fluidity of the water-absorbing material differently from what may happen when the absorbent body 1 is manufactured by disposing the absorbent sheet Sa on the water-absorbing fibers and manufacture a hybrid structure absorbent body 1 in a desired shape with high precision.

In the apparatuses 1000A-1000G for manufacturing the absorbent body 1, the absorbent sheet Sa and the absorbent fluff Fa are superimposed such that the channel area A2 of the absorbent sheet Sa and the channel part Fa2 of the absorbent fluff Fa overlap. Therefore, in the absorbent body 1, diffusion of bodily fluids through the channels C (the channel area A2 of the absorbent sheet Sa and the channel part Fa2 of the absorbent fluff Fa) can be facilitated and the absorption performance of the absorbent body 1 can be improved.

(3-2)
In the apparatuses 1000A-1000G for manufacturing the absorbent body 1, the absorbent sheet formation unit 100 has a second drum 160. The second drum 160 is disposed downstream of the first drum 150, which serves as a first suction conveyer in the conveyance direction of the absorbent sheet Sa and adjacent to the first drum 150. The absorbent sheet Sa is wrapped around the second drum 160. The press includes at least one of a first absorbent-part-press 170 and a first channel-press 190a. The first absorbent-part-press 170 sandwiches and presses the spraying area A1 of the absorbent sheet Sa. The first channel-press 190a presses the channel area A2 of the absorbent sheet Sa. The first absorbent-part-press 170 has a flat roll 172 serving as one example of a first pressing member. The first absorbent-part-press 170 pushes the flat roll 172, in a sate where the absorbent sheet Sa is wrapped around the second drum 160 between the flat roll 172 and the second drum 160, via the absorbent sheet Sa, against the second drum 160 functioning as part of the first absorbent-part-press 170. The first channel-press 190a has a second pressing member. The first channel-press 190a pushes the pattern roll 192a, in a state where the absorbent sheet Sa is wrapped around the second drum 160 between the pattern roll 192a and the second drum 160, via the channel area A2 of the absorbent sheet Sa, against the second drum 160 functioning as part of the first channel-press 190a.

In the apparatuses 1000A-1000G for manufacturing the absorbent body 1, the first absorbent-part-press 170 or the first channel-press 190a presses a predetermined location of the absorbent sheet Sa between the device and the second drum 160 disposed adjacent to the first drum 150, in other words, immediately after the absorbent sheet Sa leaves the first drum 150, to adjust the shape of the absorbent sheet Sa. Therefore, the apparatuses 1000A-1000G for manufacturing the absorbent body 1 can form an absorbent sheet Sa in a desired shape with high precision.

In the apparatuses 1000A-1000G for manufacturing the absorbent body 1, since the absorbent sheet Sa can be pressed while being wrapped around the second drum 160 in a state in which movement of the water-absorbing material is suppressed, after the pressing process, an absorbent material accumulation pattern can be formed that is less susceptible to the effects of vibrations and the like when the absorbent sheet Sa is conveyed downstream.

(3-3)
In the apparatuses 1000A-1000G for manufacturing the absorbent body 1 (excluding the manufacturing device illustrated in FIG. 9), the press further includes a second channel-press 190. The second channel-press 190 is disposed between the second drum 160 and the fiber-stacking unit 300 in the conveyance direction of the absorbent sheet Sa. The second channel-press 190 presses the channel area A2 of the absorbent sheet Sa. The absorbent sheet formation unit 100 further has a second suction conveyer 180. The second suction conveyer 180 is disposed between the second drum 160 and the second channel-press 190 in the conveyance direction of the absorbent sheet Sa. The second suction conveyer 180 has a horizontal second conveying surface 182a having a suction function, and conveys the absorbent sheet Sa while suctioning the absorbent sheet Sa to the second conveying surface 182a. The second conveying surface 182a of the second suction conveyer 180 extends from the second drum 160 to the second channel-press 190. In other words, the conveyor belt 182 of the second suction conveyer 180 extends from a position adjacent to the second drum 160 to a position adjacent to the second channel-press 190.

In the apparatuses 1000A-1000G for manufacturing the absorbent body 1 (excluding the manufacturing device illustrated in FIG. 9), at least one of the first absorbent-part-press 170 and the first channel-press 190a presses the absorbent sheet Sa to adjust the shape of the absorbent sheet Sa, and the absorbent sheet Sa is conveyed to the second channel-press 190 while movement of the water-absorbing material is suppressed by suction conveyance, whereupon the channel area A2 of the absorbent sheet Sa is further pressed by the second channel-press 190. Therefore, in the apparatuses 1000A-1000G for manufacturing the absorbent body 1 (excluding the manufacturing device illustrated in FIG. 9), a channel area A2 contributing to the diffusion of bodily fluids can be formed with high precision at the desired position on the absorbent sheet Sa.

(3-4)
In the apparatus 1000A for manufacturing an absorbent body 1 illustrated in FIG. 3B, the press of the absorbent sheet formation unit 100 includes a first channel-press 190a and a second channel-press 190. Though not illustrated, the press may include the first channel-press 190a and the second channel-press 190 in the manufacturing apparatuses 1000B-1000G as well.

The first channel-press 190a presses the channel area A2 of the absorbent sheet Sa. The second channel-press 190 is disposed between the second drum 160 and the fiber-stacking unit 300 in the conveyance direction of the absorbent sheet Sa. The second channel-press 190 presses the channel area A2 of the absorbent sheet Sa. The first channel-press 190a has a pattern roll 192a. The first channel-press 190a pushes the pattern roll 192a, in a state where the absorbent sheet Sa is wrapped around the second drum 160 between the pattern roll 192a and the second drum 160, via the channel area A2 of the absorbent sheet Sa, against the second drum 160 functioning as part of the first channel-press 190a. At least one of the first channel-press 190a and the second channel-press 190 heats the absorbent sheet Sa.

In the manufacturing apparatus 1000A illustrated in FIG. 3B, the permeability of the adhesive used to fix the water-absorbing material can be improved by heating, making it easier to dispose the water-absorbing material at the desired position between the first sheet S1 and the second sheet S2 of the absorbent sheet Sa.

Even when only one of the first channel-press 190a and the second channel-press 190 is provided as the press, it is preferable that the channel-presses 190a and 190 press the absorbent sheet Sa while heating the sheet.

(3-5)
In the apparatuses 1000A-1000G for manufacturing the absorbent body 1 (excluding the manufacturing device illustrated in FIG. 9), the press includes the first absorbent-part-press 170 and the second channel-press 190. The first absorbent-part-press 170 sandwiches and presses the spraying area A1 of the absorbent sheet Sa. The second channel-press 190 is disposed downstream of the second drum 160 in the conveyance direction of the absorbent sheet Sa. The second channel-press 190 presses the channel area A2 of the absorbent sheet Sa. The first absorbent-part-press 170 has a flat roll 172 serving as an example of a first pressing member. The first absorbent-part-press 170 pushes the flat roll 172, in a state where the absorbent sheet Sa is wrapped around the second drum 160 between the flat roll 172 and the second drum 160, via the absorbent sheet Sa, against the second drum 160 functioning as part of the first absorbent-part-press 170. The first absorbent-part-press 170 heats the absorbent sheet Sa from one side in the thickness direction of the absorbent sheet Sa. The second channel-press 190 heats the absorbent sheet Sa from the other side in the thickness direction. Though not a configuration provided by way of limitation, for example, specifically, the flat roll 172 of the first absorbent-part-press 170 and the roll 194 (a flat roll) of the second channel-press both have a heating function.

In the apparatuses 1000A-1000G for manufacturing the absorbent body 1 (excluding the manufacturing device illustrated in FIG. 9), the absorbent sheet Sa can be heated from either side of the first sheet S1 and the second sheet S2 that sandwich the water-absorbing material. Therefore, it becomes easier for the adhesive applied to both the first sheet S1 and the second sheet S2 to permeate, and the water-absorbing material sandwiched between the first sheet S1 and the second sheet S2 can be held in place more stably.

(3-6)
The apparatuses 1000A-1000G for manufacturing the absorbent body 1 are each also provided with a layered-body-press 500. The layered-body-press 500 presses a layered body L which is conveyed and in which the absorbent fluff Fa is laid over the absorbent sheet Sa. The layered-body-press 500 includes at least one of a second absorbent-part-press 510, a third channel-press 520, and an edge-press 530. The second absorbent-part-press 510 presses an entirety of the conveyed layered body L. The third channel-press 520 presses the portion of the conveyed layered body L where the channel part Fa2 of the absorbent fluff Fa and the channel area A2 of the absorbent sheet Sa overlap. The edge-press 530 presses the conveyed layered body L along a direction orthogonal to the conveyance direction of the layered body L. Specifically, the edge-press 530 presses the layered body L such that when the layered body L is cut by the downstream cutter 600 into individual absorbent bodies 1, the absorbent bodies 1 are pressed at both ends in the conveyance direction (longitudinal direction).

By pressing the layered body L, the apparatuses 1000A-1000G for manufacturing the absorbent body 1 can manufacture absorbent bodies in a desired shape with high precision.

(3-7)
In the apparatuses 1000A-1000G for manufacturing the absorbent body 1, the layered-body-press 500 includes at least a third channel-press 520. The third channel-press 520 heats the layered body L.

In the apparatuses 1000A-1000G for manufacturing the absorbent body 1, the permeability of the adhesive used to fix the water-absorbing material can be improved by heating. Therefore, it becomes easier to dispose the water-absorbing material at the desired position between the first sheet S1 and the second sheet S2 in the absorbent body 1.

(3-8)
In the apparatuses 1000E and 1000F for manufacturing the absorbent body 1, the fiber-stacking unit 300 includes a gear stretcher 327 that subjects the third sheet S3 to gear stretching. The gear stretcher 327 may be provided in the apparatuses for manufacturing the absorbent body 1 of the other embodiments.

In the apparatuses 1000E and 1000F for manufacturing the absorbent body 1, it is easy to manufacture the absorbent body 1 in a desired shape because the third sheet S3 is likely to deform. Specifically, due to the third sheet S3 being more likely to deform, the third sheet S3 supplied to the fiber-stacking drum 310 is more likely to align with the recess 314 for fiber stacking in the outer peripheral surface 312 of the fiber-stacking drum 310, and it is it is easy to manufacture the absorbent body 1 in a desired shape.

(3-9)
The method for manufacturing the absorbent body 1 of the first to seventh embodiments is a method for manufacturing a hybrid structure absorbent body including a water-absorbing material layer and a stacked fiber layer. The method for manufacturing the absorbent body includes an absorbent sheet formation process, a pressing process, an absorbent fluff formation process, and a stacking process. In the absorbent sheet formation process, a water-absorbing material is sprayed between a first sheet and a second sheet, and an absorbent sheet Sa is formed. A spraying area A1 where the water-absorbing material is sprayed and a channel area A2 where the water-absorbing material is not sprayed are formed between the first sheet S1 and the second sheet S2 of the absorbent sheet Sa. In the pressing process, the conveyed absorbent sheet Sa is pressed. In the absorbent fluff formation process, an absorbent fluff Fa, which has a stacked fiber part Fa1 in which fibers are stacked and a channel part Fa2 in which the fibers are not stacked, is formed. In the stacking process, the absorbent fluff Fa is stacked on the absorbent sheet Sa, which is conveyed while being sucked. In the stacking process, the channel part Fa2 of the absorbent fluff Fa is laid over the channel area A2 of the absorbent sheet Sa.

In the method for manufacturing the absorbent body 1 of the first to seventh embodiments, the absorbent sheet Sa, in which the position of the water-absorbing material is likely to shift, is conveyed while being sucked, and the absorbent fluff Fa is superimposed on the absorbent sheet Sa. Therefore, in the method for manufacturing the absorbent body 1 of the first to seventh embodiments, the fluidity of the water-absorbing material can be suppressed differently from what may happen when the absorbent body 1 is manufactured by disposing the absorbent sheet Sa on the water-absorbing fibers, and a hybrid structure absorbent body 1 having a desired shape can be manufactured with high precision.

In the method for manufacturing the absorbent body 1 of the first to seventh embodiments, the absorbent sheet Sa and the absorbent fluff Fa are superimposed together such that the channel area A2 of the absorbent sheet Sa and the channel part Fa2 of the absorbent fluff Fa overlap. Therefore, in the absorbent body 1, the diffusion of bodily fluids through a channel (the channel area A2 of the absorbent sheet Sa and the channel part Fa2 of the absorbent fluff Fa) can be facilitated and the absorption performance of the absorbent body can be improved.

### <Additional remarks>

Finally, the technical ideas that can be understood from the above embodiments shall be added below.

The apparatus for manufacturing an absorbent body of a first aspect is an apparatus for manufacturing a hybrid structure absorbent body having a water-absorbing material absorbent layer and a stacked fiber absorbent layer including at least fluff. The apparatus for manufacturing the absorbent body comprises an absorbent sheet formation unit and a fiber-stacking unit. The absorbent sheet formation unit forms an absorbent sheet in which a water-absorbing material is sprayed between a first sheet and a second sheet. The fiber-stacking unit stacks fibers to form an absorbent fluff including at least fluff, and stacks the absorbent fluff on the absorbent sheet, which is conveyed while being sucked. A spraying area and a channel area are formed between the first sheet and the second sheet of the absorbent sheet. The water-absorbing material is sprayed in the spraying area. The water-absorbing material is not sprayed in the channel area. The absorbent fluff has a stacked fiber part and a channel part. Fibers are stacked in the stacked fiber part. Fibers are not stacked in the channel part. The absorbent sheet formation unit has a first sheet supplier, a first suction conveyer, a water-absorbing material supplier, a second sheet supplier, and a press. The first sheet supplier supplies the first sheet. The first suction conveyer has a first conveying surface having a suction function, and conveys the first sheet while suctioning the first sheet on the first conveying surface. The water-absorbing material supplier supplies the water-absorbing material onto the first sheet conveyed by the first suction conveyer. The second sheet supplier supplies the second sheet and covers the water-absorbing material on the first sheet with the second sheet to form the absorbent sheet. The press presses the conveyed absorbent sheet. The fiber-stacking unit has a fiber supplier, a fiber-stacking drum, and a third sheet supplier. The fiber supplier supplies the fibers. The fiber-stacking drum has a suction function and causes the fibers supplied by the fiber supplier to be stacked to form the absorbent fluff. The third sheet supplier supplies a third sheet disposed on a side of the absorbent fluff opposite from the absorbent sheet such that the absorbent fluff is sandwiched between the third sheet and the absorbent sheet. The fiber-stacking unit lays the channel part of the absorbent fluff over the channel area of the absorbent sheet when stacking the absorbent fluff on the absorbent sheet.

In the apparatus for manufacturing an absorbent body of the first aspect, the absorbent sheet, in which the water-absorbing material is likely to shift position, is conveyed by suction, and the absorbent fluff is laid over the absorbent sheet. Therefore, the fluidity of the water-absorbing material can be suppressed differently from what may happen when the absorbent body is manufactured by disposing the absorbent sheet on the water-absorbing fibers, and a hybrid structure absorbent body in a desired shape can be manufactured with high precision.

In addition, in the apparatus for manufacturing the absorbent body of the first aspect, the absorbent sheet and the absorbent fluff are superimposed together such that the channel area of the absorbent sheet and the channel part of the absorbent fluff overlap. Therefore, in the absorbent body, the diffusion of bodily fluids through a channel (the channel area of the absorbent sheet and the channel part of the absorbent fluff) can be facilitated and the absorption performance of the absorbent body can be improved.

The apparatus for manufacturing an absorbent body of a second aspect is the apparatus for manufacturing the absorbent body of the first aspect, wherein the absorbent sheet formation unit further has a second drum. The second drum is disposed downstream of the first suction conveyer in the conveyance direction of the absorbent sheet and adjacent to the first suction conveyer. The absorbent sheet is wrapped around the second drum. The press includes at least one of a first absorbent-part-press and a first channel-press. The first absorbent-part-press sandwiches and presses the spraying area of the absorbent sheet. The first channel-press presses the channel area of the absorbent sheet. The first absorbent-part-press has a first pressing member. The first absorbent-part-press pushes the first pressing member, in a state where the absorbent sheet is wrapped around the second drum between the first pressing member and the second drum, via the absorbent sheet, against the second drum functioning as part of the first absorbent-part-press. The first channel-press has a second pressing member. The first channel-press pushes the second pressing member, in a state where the absorbent sheet is wrapped around the second drum between the second pressing member and the second drum, via the channel area of the absorbent sheet, against the second drum functioning as part of the first channel-press.

In the apparatus for manufacturing the absorbent body of the second aspect, the first absorbent-part-press or the first channel-press presses a predetermined location of the absorbent sheet between the device and the second drum disposed adjacent to the first suction conveyer, in other words, immediately after the absorbent sheet leaves the first suction conveyer, to adjust the shape of the absorbent sheet. Therefore, the apparatus for manufacturing the absorbent body of the second aspect can precisely form an absorbent sheet in a desired shape.

In addition, in the apparatus for manufacturing the absorbent body of the second aspect, since the absorbent sheet can be pressed while being wrapped around the second drum in a state in which movement of the water-absorbing material is suppressed, after the pressing process, an absorbent material accumulation pattern can be formed that is less susceptible to the effects of vibrations and the like when the absorbent sheet is conveyed downstream.

The apparatus for manufacturing the absorbent body of a third aspect is the apparatus for manufacturing an absorbent body of the second aspect, wherein the press further includes a second channel-press. The second channel-press is disposed between the second drum and the fiber-stacking unit in the conveyance direction of the absorbent sheet. The second channel-press presses the channel area of the absorbent sheet. The absorbent sheet formation unit further has a second suction conveyer. The second suction conveyer is disposed between the second drum and the second channel-press in the conveyance direction of the absorbent sheet. The second suction conveyer has a horizontal second conveying surface having a suction function, and conveys the absorbent sheet while suctioning the absorbent sheet to the second conveying surface. The second conveying surface of the second suction conveyer extends from the second drum to the second channel-press.

In the apparatus for manufacturing the absorbent body of the third aspect, at least one of the first absorbent-part-press and the first channel-press presses the absorbent sheet to adjust the shape of the absorbent sheet, and the absorbent sheet is conveyed to the second channel-press while movement of the water-absorbing material is suppressed by suction conveyance, whereupon the channel area of the absorbent sheet is further pressed by the second channel-press. Therefore, in the apparatus for manufacturing an absorbent body of the third aspect, a channel area contributing to the diffusion of bodily fluids can be formed with high precision at the desired position on the absorbent sheet.

The apparatus for manufacturing the absorbent body of a fourth aspect is the apparatus for manufacturing the absorbent body of any of the first to third aspects, wherein the absorbent sheet formation unit further has a second drum. The second drum is disposed downstream of the first suction conveyer in the conveyance direction of the absorbent sheet, and adjacent to the first suction conveyer. The absorbent sheet is wrapped around the second drum. The press includes a first channel-press and a second channel-press. The first channel-press presses the channel area of the absorbent sheet. The second channel-press is disposed between the second drum and the fiber-stacking unit in the conveyance direction of the absorbent sheet. The second channel-press presses the channel area of the absorbent sheet. The first channel-press has a second pressing member. The first channel-press pushes the second pressing member, in a state where the absorbent sheet is wrapped around the second drum between the second pressing member and the second drum, via the channel area of the absorbent sheet, against the second drum functioning as part of the first channel-press. At least one of the first channel-press and the second channel-press heats the absorbent sheet.

In the apparatus for manufacturing an absorbent body of the fourth aspect, the permeability of the adhesive used to fix the water-absorbing material can be improved by heating. Therefore, it becomes easier to dispose the water-absorbing material at the desired position between the first sheet and the second sheet of the absorbent sheet.

The apparatus for manufacturing the absorbent body of a fifth aspect is the apparatus for manufacturing the absorbent body of any of the first to fourth aspects, wherein the absorbent sheet formation unit further has a second drum. The second drum is disposed downstream of the first suction conveyer in the conveyance direction of the absorbent sheet, and adjacent to the first suction conveyer. The absorbent sheet is wrapped around the second drum. The press includes a first absorbent-part-press and a second channel-press. The first absorbent-part-press sandwiches and presses the spraying area of the absorbent sheet. The second channel-press is disposed downstream of the second drum in the conveyance direction of the absorbent sheet. The second channel-press presses the channel area of the absorbent sheet. The first absorbent-part-press has a first pressing member. The first channel-press pushes the first pressing member, in a state where the absorbent sheet is wrapped around the second drum between the first pressing member and the second drum, via the absorbent sheet, against the second drum functioning as part of the first absorbent-part-press. The first absorbent-part-press heats the absorbent sheet from one side in the thickness direction of the absorbent sheet. The second channel-press heats the absorbent sheet from the other side in the thickness direction.

In the apparatus for manufacturing an absorbent body of the fifth aspect, the absorbent sheet can be heated from either side of the first sheet and the second sheet that sandwich the water-absorbing material. Therefore, it becomes easier for the adhesive applied to both the first sheet and the second sheet to permeate, and the water-absorbing material sandwiched between the first sheet and the second sheet can be held in place more stably.

The apparatus for manufacturing the absorbent body of a sixth aspect is the apparatus for manufacturing the absorbent body of any of the first to fifth aspects, further comprising a layered-body-press. The layered-body-press presses a conveyed layered body in which the absorbent fluff is laid over the absorbent sheet. The layered-body-press includes at least one of a second absorbent-part-press, a third channel-press, and an edge-press. The second absorbent-part-press presses an entirety of the conveyed layered body. The third channel-press presses the portion of the conveyed layered body where the channel part of the absorbent fluff and the channel area of the absorbent sheet overlap. The edge-press presses the conveyed layered body along a direction orthogonal to the conveyance direction of the layered body.

By pressing the layered body, the apparatus for manufacturing the absorbent body of the sixth aspect can manufacture the absorbent body in a desired shape with high precision.

The apparatus for manufacturing the absorbent body of a seventh aspect is the apparatus for manufacturing the absorbent body of the sixth aspect, wherein the layered-body-press includes at least the third channel-press. The third channel-press heats the layered body.

In the apparatus for manufacturing an absorbent body of the seventh aspect, the permeability of the adhesive used to fix the water-absorbing material can be improved by heating. Therefore, it becomes easier to dispose the water-absorbing material at the desired position between the first sheet and the second sheet in the absorbent body.

The apparatus for manufacturing the absorbent body of an eighth aspect is the apparatus for manufacturing the absorbent body of any of the first to seventh aspects, wherein the fiber-stacking unit includes a gear stretcher that subjects the third sheet to gear stretching.

In the apparatus for manufacturing the absorbent body of the eighth aspect, it is easy to manufacture the absorbent body in a desired shape because the third sheet becomes more likely to deform.

A method for manufacturing an absorbent body of a ninth aspect is a method for manufacturing a hybrid structure absorbent body having a water-absorbing material layer and a stacked fiber layer. The method for manufacturing the absorbent body comprises an absorbent sheet formation process, a pressing process, an absorbent fluff formation process, and a stacking process. In the absorbent sheet formation process, a water-absorbing material is sprayed between a first sheet and a second sheet, and an absorbent sheet is formed. A spraying area where the water-absorbing material is sprayed and a channel area where the water-absorbing material is not sprayed are formed between the first sheet and the second sheet of the absorbent sheet. In the pressing process, the conveyed absorbent sheet is pressed. In the absorbent fluff formation process, an absorbent fluff, which has a stacked fiber part in which fibers are stacked and a channel part in which the fibers are not stacked, is formed. In the stacking process, the absorbent fluff is stacked on the absorbent sheet, which is conveyed while being sucked . In the stacking process, the channel part of the absorbent fluff is laid over the channel area of the absorbent sheet.

In the method for manufacturing an absorbent body of the ninth aspect, the absorbent sheet, in which the position of the water-absorbing material is likely to shift, is conveyed while being sucked, and the absorbent fluff is superimposed on the absorbent sheet. Therefore, in the method for manufacturing the absorbent body of the ninth aspect, the fluidity of the water-absorbing material can be suppressed, differently from what may happen when the absorbent body is manufactured by disposing the absorbent sheet on the water-absorbing fibers, and a hybrid structure absorbent body having a desired shape can be manufactured with high precision.

In the method for manufacturing the absorbent body of the ninth aspect, the absorbent sheet and the absorbent fluff are superimposed together such that the channel area of the absorbent sheet and the channel part of the absorbent fluff overlap. Therefore, in the absorbent body, the diffusion of bodily fluids through a channel (the channel area of the absorbent sheet and the channel part of the absorbent fluff) can be facilitated and the absorption performance of the absorbent body can be improved.

### REFERENCE SIGNS LIST

- 1: Absorbent body
- 100: Absorbent sheet formation unit
- 110: First sheet supplier
- 120: Second sheet supplier
- 140: Water-absorbing material supplier
- 150: First drum (first suction conveyer)
- 152: First conveying surface
- 160: Second drum
- 170: First absorbent-part-press (press)
- 172: Flat roll (first pressing member)
- 180: Second suction conveyer
- 182a: Second conveying surface
- 190: Second channel-press (press)
- 190a: First channel-press (press)
- 192a: Pattern roll (second pressing member)
- 220: Third suction conveyer
- 222a: Third conveying surface
- 300: Fiber-stacking unit
- 310: Fiber-stacking drum
- 320: Third sheet supplier
- 327: Gear stretcher
- 330: Fiber supplier
- 500: Layered-body-press
- 510: Second absorbent-part-press
- 520: Third channel-press
- 530: Edge-press
- A1: Spraying area
- A2: Channel area
- Fa: Absorbent fluff
- Fa1: Stacked fiber part
- Fa2: Channel part
- L: Layered body
- S1: First sheet
- S2: Second sheet
- Sa: Absorbent sheet

## Claims

1. An apparatus for manufacturing a hybrid structure absorbent body having a water-absorbing material absorbent layer and a stacked fiber absorbent layer including at least fluff, the manufacturing apparatus comprising:
an absorbent sheet formation unit configured to form an absorbent sheet in which a water-absorbing material is sprayed between a first sheet and a second sheet; and
a fiber-stacking unit configured to stack fibers to form an absorbent fluff including at least fluff, and stack the absorbent fluff on the absorbent sheet, which is conveyed while being sucked,
a spraying area where the water-absorbing material is sprayed and a channel area where the water-absorbing material is not sprayed being formed between the first sheet and the second sheet of the absorbent sheet,
the absorbent fluff having a stacked fiber part where the fibers are stacked and a channel part where the fibers are not stacked,
the absorbent sheet formation unit having
a first sheet supplier configured to supply the first sheet,
a first suction conveyer including a first conveying surface having a suction function and configured to convey the first sheet while suctioning the first sheet on the first conveying surface,
a water-absorbing material supplier configured to supply the water-absorbing material onto the first sheet conveyed by the first suction conveyer,
a second sheet supplier configured to supply the second sheet and cover the water-absorbing material on the first sheet with the second sheet to form the absorbent sheet, and
a press configured to press the conveyed absorbent sheet,
the fiber-stacking unit having
a fiber supplier configured to supply the fibers,
a fiber-stacking drum having a suction function and configured to cause the fibers supplied by the fiber supplier to be stacked to form the absorbent fluff, and
a third sheet supplier configured to supply a third sheet to be disposed on a side of the absorbent fluff opposite from the absorbent sheet such that the absorbent fluff is sandwiched between the third sheet and the absorbent sheet, and
the fiber-stacking unit configured to lay the channel part of the absorbent fluff over the channel area of the absorbent sheet when stacking the absorbent fluff on the absorbent sheet.

2. The apparatus for manufacturing the absorbent body according to claim 1, wherein
the absorbent sheet formation unit further has a second drum, which is disposed downstream of the first suction conveyer in a conveyance direction of the absorbent sheet and adjacent to the first suction conveyer, the absorbent sheet being wrapped around the second drum,
the press includes at least one of
a first absorbent-part-press configured to sandwich and press the spraying area of the absorbent sheet, and
a first channel-press configured to press the channel area of the absorbent sheet,
wherein
the first absorbent-part-press has a first pressing member, and the first absorbent-part-press is configured to push the first pressing member, in a state where the absorbent sheet is wrapped around the second drum between the first pressing member and the second drum, via the absorbent sheet, against the second drum functioning as part of the first absorbent-part-press; and
the first channel-press has a second pressing member, and the first channel-press is configured to push the second pressing member, in a state where the absorbent sheet is wrapped around the second drum between the second pressing member and the second drum, via the channel area of the absorbent sheet, against the second drum functioning as part of the first channel-press.

3. The apparatus for manufacturing the absorbent body according to claim 2, wherein
the press further includes a second channel-press that is disposed between the second drum and the fiber-stacking unit in the conveyance direction of the absorbent sheet, and is configured to press the channel area of the absorbent sheet,
the absorbent sheet formation unit further has a second suction conveyer that includes a horizontal second conveying surface having a suction function and that is disposed between the second drum and the second channel-press in the conveyance direction of the absorbent sheet, and is configured to convey the absorbent sheet while suctioning the absorbent sheet on the second conveying surface, and
the second conveying surface of the second suction conveyer extends from the second drum to the second channel-press.

4. The apparatus for manufacturing the absorbent body according to any one of claims 1 to 3, wherein:
the absorbent sheet formation unit further has a second drum, which is disposed downstream of the first suction conveyer in the conveyance direction of the absorbent sheet and adjacent to the first suction conveyer, and the absorbent sheet being wrapped around the second drum,
the press includes
a first channel-press configured to press the channel area of the absorbent sheet, and
a second channel-press being disposed between the second drum and the fiber-stacking unit in the conveyance direction of the absorbent sheet and configured to press the channel area of the absorbent sheet,
wherein
the first channel-press has a second pressing member, and the first channel-press is configured to push the second pressing member, in a state where the absorbent sheet is wrapped around the second drum between the second pressing member and the second drum, via the channel area of the absorbent sheet, against the second drum functioning as part of the first channel-press, and
at least one of the first channel-press and the second channel-press is configured to heat the absorbent sheet.

5. The apparatus for manufacturing an absorbent body according to any one of claims 1 to 4, wherein
the absorbent sheet formation unit further has a second drum, which is disposed downstream of the first suction conveyer in a conveyance direction of the absorbent sheet and adjacent to the first suction conveyer, the absorbent sheet being wrapped and around the second drum,
the press includes
a first absorbent-part-press configured to sandwich and press the spraying area of the absorbent sheet, and
a second channel-press being disposed downstream of the second drum in the conveyance direction of the absorbent sheet and configured to press the channel area of the absorbent sheet,
wherein
the first absorbent-part-press has a first pressing member, and the first absorbent-part-press is configured to push the first pressing member, in a state where the absorbent sheet is wrapped around the second drum between the first pressing member and the second drum, via the absorbent sheet, against the second drum functioning as part of the first absorbent-part-press,
the first absorbent-part-press is configured to heat the absorbent sheet from one side of the absorbent sheet in a thickness direction; and
the second channel-press is configured to heat the absorbent sheet from the other side in the thickness direction.

6. The apparatus for manufacturing the absorbent body according to any one of claims 1 to 5, further comprising
a layered-body-press configured to press a conveyed layered body in which the absorbent fluff is laid over the absorbent sheet,
wherein
the layered-body-press includes at least one of
a second absorbent-part-press configured to press an entirety of the conveyed layered body,
a third channel-press configured to press a portion of the conveyed layered body where the channel part of the absorbent fluff and the channel area of the absorbent sheet overlap, and
an edge-press configured to press the conveyed layered body along a direction orthogonal to the conveyance direction of the layered body.

7. The apparatus for manufacturing the absorbent body according to claim 6, wherein
the layered-body-press includes at least the third channel-pressing apparatus, and
the third channel-press is configured to heat the layered body.

8. The apparatus for manufacturing the absorbent body according to any one of claims 1 to 7, wherein
the fiber-stacking unit further includes a gear stretcher configured to stretch the third sheet.

9. A method for manufacturing a hybrid structure absorbent body having a water-absorbing material absorbent layer and a stacked fiber absorbent layer including at least fluff, the method comprising:
a process of forming an absorbent sheet in which a spraying area where a water-absorbing material is sprayed and a channel area where the water-absorbing material is not sprayed are provided between a first sheet and a second sheet by spraying the water-absorbing material between the first sheet and the second sheet;
a process of pressing the conveyed absorbent sheet;
a process of forming an absorbent fluff including a stacked fiber part where fibers are stacked and a channel part where the fibers are not stacked; and
a process of stacking the absorbent fluff on the absorbent sheet, which is conveyed while being sucked,
the channel part of the absorbent fluff being laid over the channel area of the absorbent sheet in the process of stacking the absorbent fluff on the absorbent sheet.
